# EUROPÄISCHE PATENTSCHRIFT

(11) **EP 4 007 689 B1**
(45) Veröffentlichungstag und Bekanntmachung des Hinweises auf die Patenterteilung: **19.06.2024**
(21) Anmeldenummer: 20747426.3
(22) Anmeldetag: 03.08.2020
(51) Int. Cl.: B29C 64/112, B29C 64/245, B29C 64/343, B33Y 10/00, B33Y 30/00, B33Y 40/00, B33Y 80/00, A61L 27/50, A61L 27/38, A61L 27/54, C12N 5/071, C12N 5/00

(54) **VERFAHREN UND STEUEREINHEIT ZUM 3D DRUCK VON VASKULARISIERTEN GEWEBEN UND ORGANEN**
METHOD AND CONTROL UNIT FOR 3D PRINTING OF VASCULARIZED TISSUES AND ORGANS
PROCÉDÉ ET UNITÉ DE COMMANDE POUR L D'IMPRESSION 3D DE TISSUS ET DE ORGANES VASCULARISÉS

(30) Priorität: 02.08.2019 EP 19189755
(43) Veröffentlichungstag der Anmeldung: 08.06.2022
(73) Patentinhaber: Technische Universität Berlin, 10623 Berlin (DE)
(72) Erfinder: SCHÄFER, Konstanze, 10405 Berlin (DE); SALOMON, Andreas, 13505 Berlin (DE)
(74) Vertreter: Hertin und Partner Rechts- und Patentanwälte PartG mbB
(86) Internationale Anmeldenummer: PCT/EP2020/071804
(87) Internationale Veröffentlichungsnummer: WO 2021/023708

(56) Entgegenhaltungen:
- WO-A1-2018/035282
- US-A1- 2011 076 734

## Beschreibung

Die Erfindung betrifft ein 3D-Druckverfahren zur Herstellung von vaskulären Strukturen aufweisenden Geweben und Organen gemäß dem Oberbegriff des Anspruchs 1 sowie eine Steuereinheit gemäß dem Oberbegriff des Anspruchs 10.

### Einleitung:

WO 2018/035282 A1, dem Oberbegriff der Ansprüche 1 und 10 entsprechend, ist ein Beispiel des Standes der Technik und offenbart Methoden, Systeme und Bibliotheken zur Verwendung beim Tintenstrahldrucken von Biostrukturen mit vorbestimmten zwei-(2D) und dreidimensionalen (3D) Mustern von Zellen, extrazellulärer Matrix, organischen und anorganischen Komponenten und deren Kombination. Insbesondere bezieht sich die Veröffentlichung auf Verfahren, Systeme und Bibliotheken zur Verwendung beim direkten Tintenstrahldruck von Biostrukturen unter Verwendung von Drop-on-Demand, die eine vorbestimmte dreidimensionale Struktur mit lebensfähigen Zellen in einem nicht zufälligen 2D- und 3D-Muster aufweisen, wobei die Zellen von verschiedenen Typen sein können.

Herkömmliche 3D-Drucksysteme besitzen aufgrund ihrer spezifischen Merkmale und der damit einhergehenden Vor- und Nachteile nicht die erforderlichen Eigenschaften, um lebensfähige größere Gewebe und Organe erzeugen zu können.

Um ein größeres lebensfähiges Gewebe oder Organ mittels 3D-Druck herzustellen, muss insbesondere die Ernährung der gedruckten Zellen gewährleistet sein. Da die Ernährung der Zellen durch Diffusion nur über die Wegstrecke von ca. 500 µm erfolgen kann, ist ein ebenfalls gedrucktes Blutgefäßsystem erforderlich. Zwischen den Arterien und Venen befinden sich das Kapillarsystem, dessen einzelne Kapillargefäße einen sehr geringen Durchmesser von nur ca. 10 µm besitzen und zudem aus einzelligen Zell-Lagen aufgebaut sind.

Bei den bisher bekannten 3D-Drucktechniken handelt es sich entweder um Verfahren, bei denen zwar gleichzeitig verschiedene Zelltypen in einem Schritt gedruckt werden können, die dafür aber die nötige hohe Druckauflösung nicht erreichen (z.B. Fused Deposition Modeling oder Droplet-Technologien), oder um Verfahren, die die erforderliche hohe Druckauflösung erreichen, dafür aber der selektive Druck verschiedener Zelltypen innerhalb eines Arbeitsschritts unmöglich ist (z.B. Stereolithographie-Drucktechniken).

Zusätzlich gibt es spezifische 3D-Drucksysteme wie z.B. den Laser Induced Forward Transfer, bei dem durch einen Laserstrahl einzelne Zellen aus einer Matrix herausgelöst und auf eine Kollektormatrix geleitet werden. Hier kann sowohl eine hohe Druckauflösung erreicht als auch in einem Schritt verschiedene Zelltypen gedruckt werden. Allerdings ist die dabei erreichbare Druckgeschwindigkeit so niedrig, dass diese Technik nur für extrem kleine Strukturen nützlich ist.

Ein anderes spezifisches Drucksystem ist die die Weiterentwicklung des 3D - Stereolithographie - Drucks, bei dem auch verschiedene Zelltypen gedruckt werden können. Hierzu muss insbesondere aber das zu druckenden Gewebe zwischen Reaktionswannen mit unterschiedlichen Zelltypen gewechselt werden. Dieser Schritt lässt ebenfalls nur sehr wenige Zell-Lagen zu und ist ein relativ langsamer Prozess, mit dem nur kleinere Strukturen wie Miniorgane gedruckt werden können.

Weitere spezifische 3D - Druckverfahren lassen den Raum für spätere Blutgefäße als Hohlraum im gedruckten Gewebe offen und ernähren die umliegenden Zellen zunächst mit Nährmedium. Um echte Kapillaren zu erzeugen, müssen diese Gewebe in einen Organismus überführt werden, der die Hohlräume mit Endothelzellen und diese umgebenden Muskelzellen versorgt. Dieser Schritt kann auch nachträglich in einem Inkubator erfolgen. Wenn auf diese Weise auch letztendlich Kapillaren im Gewebe erzeugt werden können, so dauert der Prozess doch mindestens Tage und trotzdem wird die natürliche Komplexität des betreffenden Gewebes nicht erreicht. Abgesehen davon können mit diesen Techniken nur verhältnismäßig große Gefäßdurchmesser erreicht werden.

Eine weitere Möglichkeit, relativ einfach aufgebaute Gewebe aus langsam wachsenden Zellen wie Knorpel oder Knochen zu erzeugen, ist der 3D-Druck von Scaffolds, die nachträglich mit Zellen besiedelt werden. In diesen langsam wachsenden Gewebetypen bleibt aufgrund des niedrigen Stoffumsatzes genügend Zeit für eine nachträgliche Ausbildung von Kapillaren innerhalb eines Organismus. Dieses Vorgehen ist aber auf Gewebetypen wie Knochen und Knorpel begrenzt. Für schneller wachsende unkomplizierte Zelltypen wie beispielsweise Leberzellen konnte gezeigt werden, dass sehr kleine Gewebebereiche erfolgreich in Leberorgane verpflanzt werden können. Diese Gewebebereiche können aber nur aus sehr kleinen Strukturen bestehen, da die Zellen sonst nicht mehr ernährt werden können.

Alle bisherigen Versuche, funktionstüchtige Gewebe oder Organe mit einem 3D-Drucker zu erzeugen, führten entweder zu sehr kleinen oder sehr langsam wachsenden Gewebeeinheiten oder zu größeren Strukturen, die aufgrund des Mangels an ausgeprägten Kapillaren nur für sehr kurze Zeit lebensfähig waren.

Es bedarf also eines 3D-Druckverfahrens, das
- einerseits innerhalb eines Arbeitsschrittes verschiedene Zelltypen selektiv drucken kann und
- das andererseits die hohe Druckauflösung von Stereolithographie-Verfahren erreichen kann.
- Zusätzlich soll das Verfahren eine Druckgeschwindigkeit besitzen, mit der der Druck von ganzen Organen real durchführbar ist.
- Außerdem sollte das neuartige Drucksystem insbesondere bereits während des Drucks neu gebildetes Gewebe über die dabei erzeugten Kapillaren mit Medium oder anderen zur Ernährung der Zellen geeigneten Flüssigkeiten durchspülen.
- Das Druckverfahren sollte insbesondere auch ein neuartiges Druckmuster bereit stellen, mit dem es möglich ist, ein Kapillarsystem zwischen arteriellen und venösen Strukturen auszubilden, in dem die darin fließende Flüssigkeit (z.B. Medium oder Blut) von arteriell nach venös strömen kann.
- Die Umsetzung des neuartigen Druckverfahrens erfordert in wesentlichen Ausführungsformen einen neuartigen Druckertisch, da herkömmliche Druckertische zum Stand der Technik nicht in der Lage sind, gedrucktes Gewebe während des Drucks mit Nährmedium zu versorgen.
- Die Umsetzung des neuartigen Druckverfahrens erfordert bevorzugt eine Druckerkopfversorgungseinheit, die die gewünschte Zelldichte sowie die erforderliche Tintenkonzentration im Drucktropfen nach Vorgaben des Druckalgorithmus mischt.

Die erfindungsgemäße Aufgabe wird durch ein neuartiges UV-vernetzendes Droplet - Verfahren gemäß Anspruch 1 gelöst, das aufgrund seiner niedrigviskosen Tinten die hochauflösenden Druckerköpfe von fotorealistischen Kunstdruckern nutzen kann und das zusätzlich durch hochspezifische, räumlich begrenzte Reaktionen im Randbereich der einzelnen Tropfen die Druckauflösung bis auf eine molekulare Ebene erhöht.

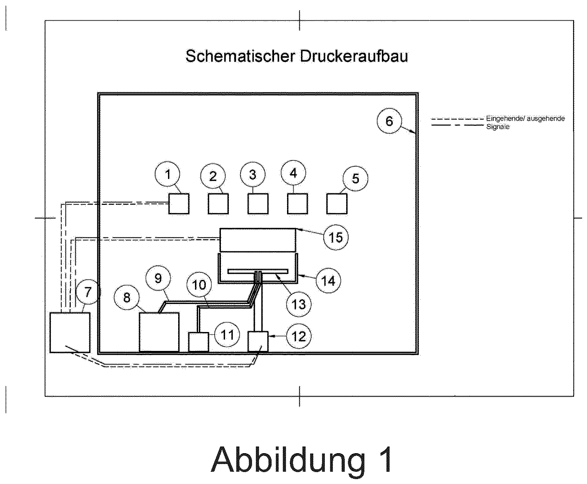

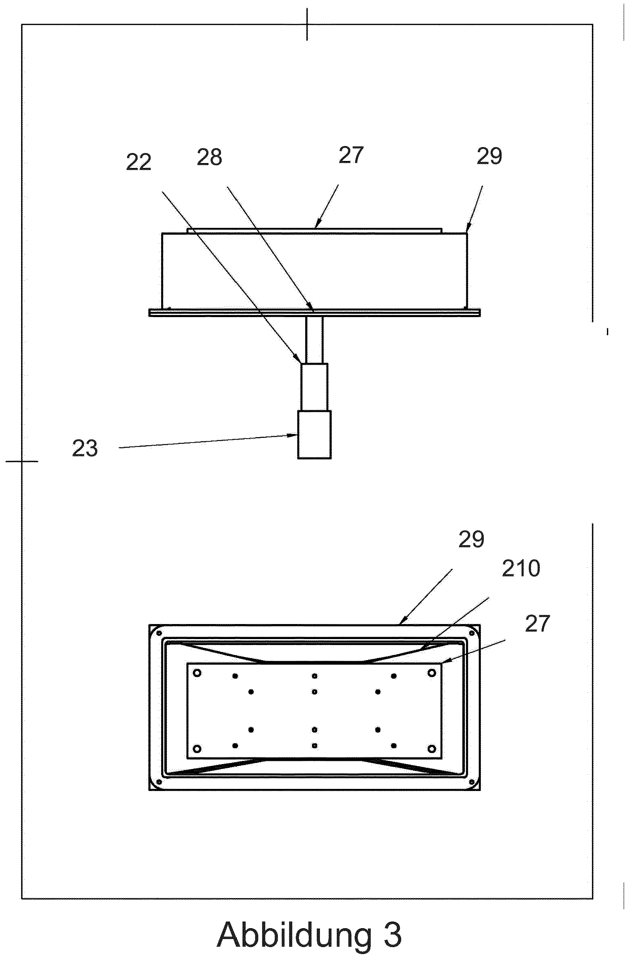

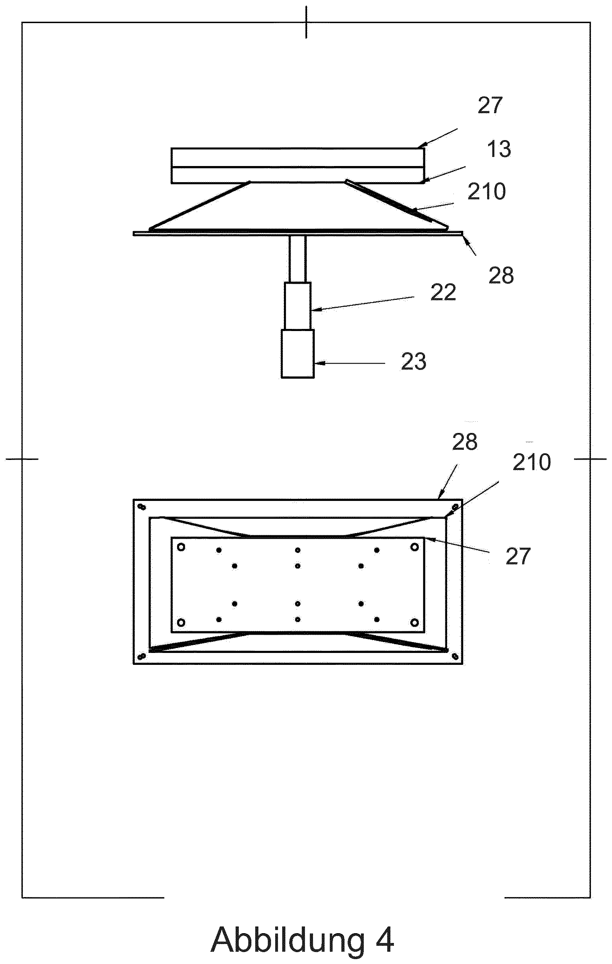

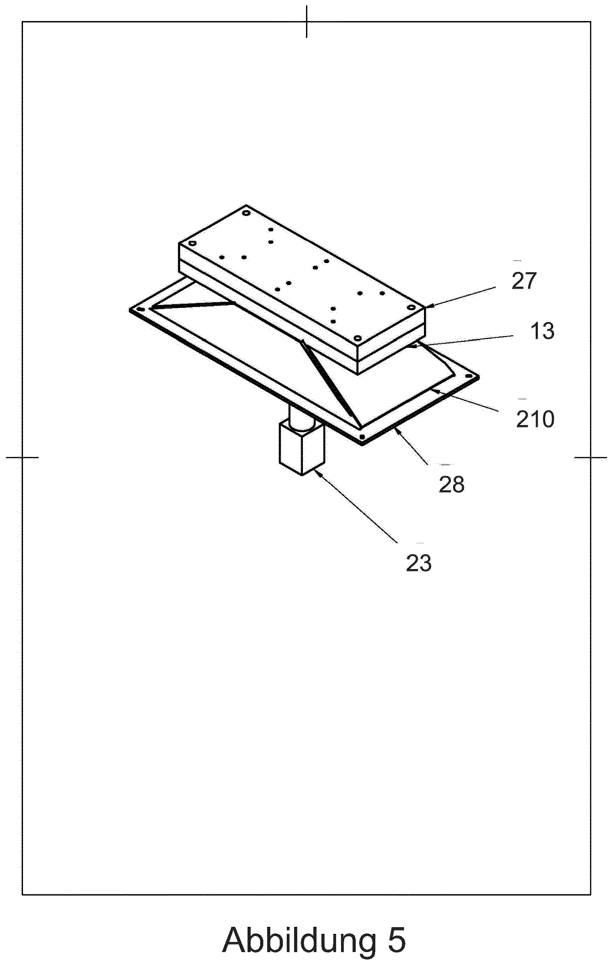

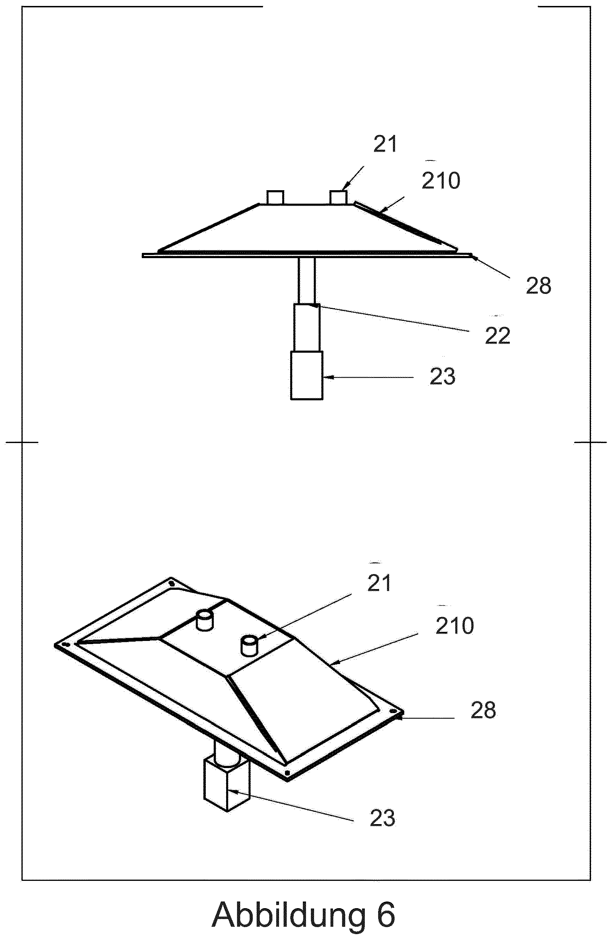

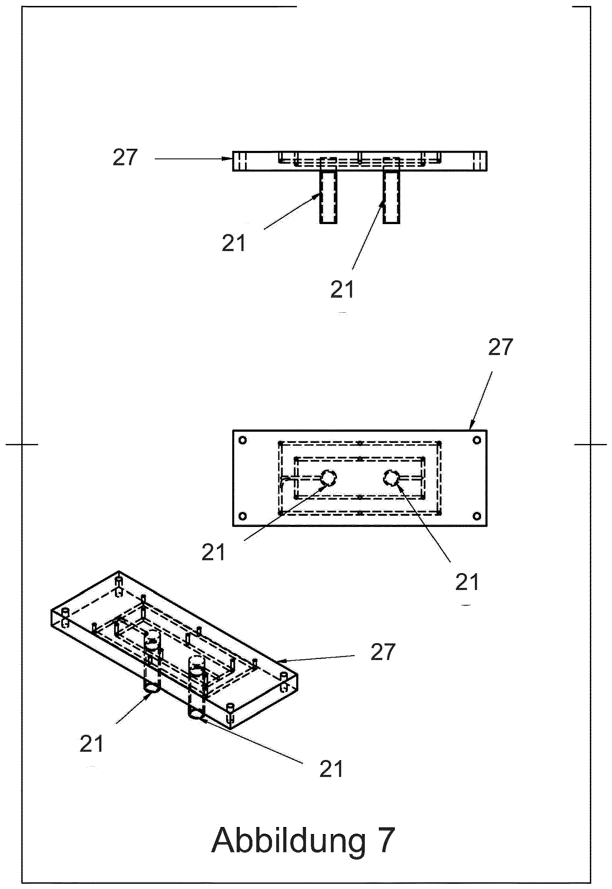

Bisherige Droplet-Verfahren zum Druck von Zellen sind Vorrichtungen, bei denen ähnlich wie durch einen Extruder relativ große Tropfen aus Hydrogel-Tinten und darin befindlichen Zellen vom Druckerkopf abgeschieden werden (thermal oder piezoelektrisch, siehe Abb. 1 und Abb.2-7). In der Regel existiert für eine Zelltyp-Tinte nur eine einzige Druckerdüse. Diese Düse hat einen relativ großen Durchmesser, da die Viskosität des noch im Solzustand befindlichen Hydrogels trotzdem relativ hoch ist (Abb. 1 und Abb.2-7). Die dadurch erzeugten Tropfen sind relativ groß und beinhalten in der Regeleine hohe Anzahl von Zellen (ca. 2000 Zellen pro Tropfen).

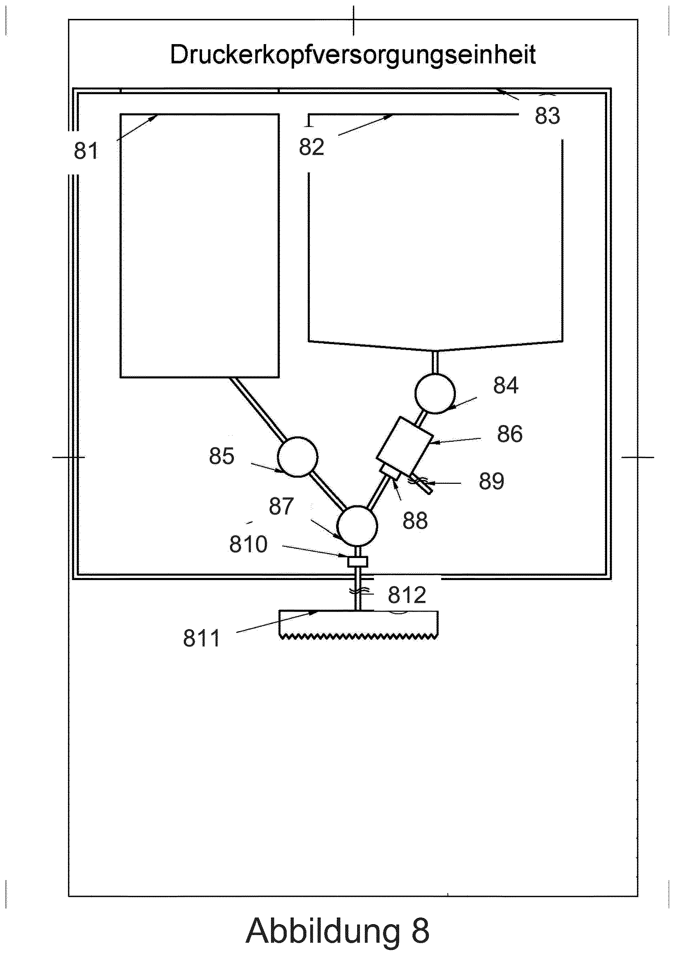

Überraschenderweise hat sich gezeigt, dass durch die Verwendung neu formulierter photosensitiver Zelltinten statt der bisher genutzten Hydrogele die Viskosität der Zelltinten - Tropfen so sehr erniedrigt werden konnte, dass diese von Druckerköpfen aus dem hochauflösenden Kunstdruckbereich verarbeitet werden können. Das war bisher nicht möglich. Dadurch sind Zelltintentropfen von wenigen Pikolitern (mit ca. 20 Zellen pro Tropfen) druckbar (Abb. 8-10). Mit dieser Druckauflösung kann man bereits Kapillaren drucken, die wesentlich kleiner im Durchmesser sind als die Kapillaren bisheriger Systeme. Zusätzlich stellte sich heraus, dass die Druckauflösung nochmals weiter erhöht werden konnte, indem man die Tinten so gestaltete, dass nur auf den Randbereich der Kapillartintentropfen begrenzt eine Vernetzungsreaktion stattfand. Das restliche Tropfenvolumen hingegen durchläuft diese Vernetzungsreaktion nicht und wird anschließend ausgespült oder auf anderem Wege beseitigt, um anschließend als Kapillarhohlraum zu fungieren. Durch diesen neuartigen Druckvorgang konnte die Druckauflösung bis zu einer Größenordnung auf einer molekularen Ebene bzw. einzelnen Zellen bzw. Zelllagen erhöht werden und hat damit die Feinheit von Stereolithographie-Systemen erreicht. Die mit diesem Verfahren druckbaren Kapillaren sind so fein wie die Kapillaren des menschlichen Organismus.

Die auf den Randbereich der Kapillartintentropfen begrenzte Vernetzung kann sowohl durch Tintenbestandteile als auch durch einen gerichteten Laserstrahl (oder eine andere gerichtete elektromagnetische Quelle) erfolgen. Die dabei ablaufenden Reaktionen können physikalischer, chemischer oder biologischer Natur sein (siehe Abschnitt Beispiele).

Während des gesamten Druckprozesses wird das entstehende Gewebe bevorzugt über die kapillaren Strukturen mit Nährmedium durchspült. Dafür wird während des Drucks ein spezifisches Druckmuster erzeugt, das die Bildung von Kapillaren zwischen zukünftigen arteriellen und venösen Strukturen organisiert.

Soll ein ganzes Organ gedruckt und später in einen Organismus oder Patienten implantiert werden, kann das Organ für einen begrenzten Zeitraum vor der Implantation in einer speziell dafür hergestellten Vorrichtung außerhalb des Körpers an den Blutkreislauf des Patienten angeschlossen werden. Durch diese Vorrichtung können die physiologischen Werte des Organs überprüft und der optimale Implantationszeitpunkt ermittelt werden.

### Stand der Technik:

In Deutschland warten bis zu 15.000 Patienten auf ein Spenderorgan. Lediglich für weniger als 1/3 von ihnen kann ein passendes Organ gefunden werden. Für Forschung und Pharmazie werden in Deutschland jährlich ca. 3 Mio. Tierversuche unternommen, die durch Alternativen überflüssig gemacht werden könnten. Ein weiteres Gebiet für den 3D-Druck von Geweben ist die regenerative Medizin, die weiterhin große Fortschritte macht.

Der 3D-Bioprint kann für diese und andere Anwendungsfelder eine Lösung herbeiführen. Durch ihn werden Zellstrukturen mit 3D-Druckern hergestellt, wobei Zellenebenen in einer additiven Fertigung aufeinandergeschichtet werden, um so komplexe dreidimensionale Strukturen zu bilden. Geeignet für das Biodrucken sind besonders Techniken wie das Fused Deposition Modeling, das Droplet-Verfahren und die Stereolithographie:
Das Fused Deposition Modeling ist ein extrusionsbasiertes Druckverfahren, das Thermoplaste und Verbundwerkstoffe sowie Keramiken und Metalle verarbeitet. Auch Zellen in Alginat- oder Gelatinegelen sind damit druckbar. Nachteilig bei diesem Verfahren ist besonders die geringe Druckauflösung. Das Dropletverfahren verarbeitet polymere Lösungen, kolloidale Suspensionen und Zellsuspensionen. Die Auflösung beim Dropletverfahren ist zwar höher als die des Fused Deposition Modeling, aber nicht ausreichend für den Druck von vaskulären Strukturen. Die Stereolithographie besitzt dagegen durch die Verwendung eines feinen Laserstrahls die höchste Auflösung, die bisher erreichbar ist. Sie verarbeitet eine viskose photoaushärtbare Polymerlösung, die einem gerichteten elektromagnetischen Strahl ausgesetzt wird, um die Lösung räumlich zu vernetzen. Ihr Nachteil liegt darin, dass immer nur ein einziger Zelltyp innerhalb einer Reaktionswanne druckbar ist. Wenn man einen weiteren Zelltyp hinzufügen möchte, muss bevorzugt das Reaktionsbad gewechselt werden.

Im Stand der Technik ist der *Inkjet Biodruck des Marktführers Organovo* beschrieben, der Tintentröpfchen mit jeweils 10.000 bis 30.000 Einzelzellen erzeugt (Quelle: Organovo). Die Firma Organovo nutzt den Biodruck insbesondere zur Herstellung von Lebergewebe. In der Regel werden in das geschädigte Organ 3D-gedruckte Gewebeteile eingepflanzt, die in ihrer Wirkungsweise die bestehende Leber unterstützen und so die Lebensdauer dieses Organs verlängern, bis ein geeigneter Spender gefunden ist.

Beim *Extrusions-Biodruck* werden Biotinten mit Hilfe von mechanischem Druck aus einer kleinen Nadel gespritzt. So wird das gesamte Zellgerüst Schicht für Schicht gedruckt. Dieses Verfahren wird beispielsweise von Biodruckern der Firma *Envisiontec GmbH* genutzt. Die Arbeitsgeschwindigkeit ist relativ hoch, leider ist die Druckauflösung aber sehr niedrig.

Die Stereolithographie wird beispielsweise von den Firmen Cellbricks und TissUse ("organ on a chip") verwendet. Sie liefert die höchste Druckauflösung, da mit Hilfe eines Laserstrahls ein Hydrogel vernetzt wird. Allerdings kann mit diesem Verfahren immer nur ein Zelltyp gedruckt werden. Um das Gewebe aus mehreren Zelltypen zusammen zu setzen, muss bevorzugt das entstehende Gewebe durch verschiedene Reaktionswannen gewechselt werden, wodurch der Prozess verlangsamt wird und das gedruckte Gewebe nur geringe Ausmaße erreicht.

Neben diesen am häufigsten verwendeten Verfahren existieren eine Reihe von Verfahren, die aus Modifizierungen hervorgegangen sind:
Einige Techniken zielen darauf ab, dass sich die sehr feinen Kapillaren (im Durchmesser bis zu 10 µm klein) zwischen den größeren gedruckten Venen und Arterien während der Zellkultur selbst bilden (Yu et al., 2014). Problematisch hierbei ist, dass das gedruckte Gewebe für längere Zeit ausgereift werden muss. Je länger das Gewebe in Zellkultur gehalten wird, desto größer wird die Wahrscheinlichkeit für Artefakte.

Ein anderer Lösungsansatz ist der Druck von schlauchförmigen Strukturen mit einem Polymer, das anschließend wieder depolymerisiert und ausgespült wird und dadurch einen Hohlraum für die Bildung eines vaskulären Systems freigeben soll (Kolesky et al., 2014; Lee et al., 2014a,b). Dieser Lösungsansatz ist aber unzureichend und unterscheidet sich zum hier offenbarten neuartigen Verfahren in zwei Punkten: 1) es handelt sich um einen Schlauch ohne Endothelzellen und feinste Verästelungen, eine längere Inkubation (mit zu erwartenden Artefakten) ist erforderlich und 2) die Druckauflösung ist viel zu niedrig, um ein echtes kapillares System erzeugen zu können.

Ein weiterer Lösungsansatz ist ein Tandem-Druck: zuerst wird mit einem Stereolithographie-System (das eine hohe Auflösung hat aber nur einen einzigen Zelltyp verdrucken kann) das vaskuläre Netzwerk gedruckt. Anschließend wird die gedruckte vaskuläre Struktur in ein anderes 3D-Drucksystem überführt (das keine gute Auflösung hat, aber verschiedene Zelltypen verdrucken kann), in dem die restlichen Gewebezellen gedruckt werden (Yu et al., 2014). Dazu muss insbesondere die gedruckte vaskuläre Struktur aus dem Reaktionsbehälter des einen 3D-Drucksystems entfernt und in den Reaktionsbehälter des anderen 3D-Drucksystems überführt werden. Besondere Unterschiede zum hier offenbarten Verfahren sind: 1) Es können keine einzelligen Zell-Lagen gedruckt werden, die Strukturen wären bei Wechsel zu fragil und 2) es werden zwei verschiedene Drucker benötigt, beim Wechsel steigt das Kontaminationsrisiko.

Um das hier offenbarte Verfahren umsetzen zu können, bedarf es in bevorzugten Ausführungsformen insbesondere eines Druckertisches, der das gedruckte Gewebe mit Nährmedium versorgen kann, damit die Zellen nicht unterversorgt sind oder absterben. Außerdem muss sich der Druckertisch bevorzugt in einem flüssigkeitsgefüllten Druckraum befinden.

Druckertische und Druckräume der herkömmlichen 3D-Druckverfahren sind hierfür nicht nutzbar. Beispielsweise haben die meisten 3D-Drucksysteme (z.B. FDN, SLS, BJ und andere) einen trockenen Druckertisch in einem trockenen Druckraum und besitzen keine Möglichkeit für eine Flüssigkeitsaufnahme.

Einige wenige 3D-Druckertypen besitzen zwar einen Flüssigkeitsgefüllten Druckraum (z.B. SLA), hier gibt es aber keinerlei Versorgungssystem für das gedruckte Gewebe mit einem zusätzlichen Flüssigkeitszulauf und Ablauf, wie er beim 3D-Druck von vaskulären Strukturen aufweisenden Geweben und Organen erforderlich ist.

Das hier offenbarte neuartige Drucksystem baut auf einem Dropletverfahren auf und unterscheidet sich in mehreren Punkten von bisherigen Droplet-Systemen, die folgend aufgeführt sind:
1) Es werden neuartige photosensitive Biotinten genutzt, die so niedrigviskos sind, dass sie die feinen Druckerköpfe von photorealistischen Kunstdruckern nicht verstopfen. Bisherige Tintentropfen enthalten ca. 2000 - 3000 Zellen und bestehen aus Hydrogelen (Organovo). Die Hydrogelbestandteile sind der Grund für die relativ großen Tropfen, da ihre Viskosität auch im Solzustand relativ hoch ist. Die im hier offenbarten Verfahren erzeugten Tröpfchen sind sehr klein, niedrigviskos und enthalten ca. 20 Zellen oder weniger. Die gedruckte Zellmasse wird dadurch erreicht, dass die Kunstdruck-Druckerköpfe statt nur einer Düse pro Tintentyp (wie bisher in Anwendung) ca. 160 Düsen oder mehr besitzen.
   Gleichzeitig besitzen die Tinten eine Formulierung, die es ermöglicht, unmittelbar zu vernetzen und dadurch ein Verlaufen der Tropfen zu verhindern. Bisher existierende Hydrogele im Dropletverfahren, die photosensitiv waren, nutzen Aushärtungen, die durch eine Lichtquelle außerhalb des Druckerkopfes ausgelöst werden und die über einen längeren Zeitraum von mehreren Minuten andauern. In dem hier offenbarten neuartigen Verfahren befindet sich die Quelle der elektromagnetischen Wellen (z.B. sichtbares Licht, IR, UV) direkt im Druckerkopf und kann sowohl starr als auch gerichtet sein.
2) Spezielle Biotinten (Kapillartinten) innerhalb des hier offenbarten neuartigen Drucksystems vernetzen nur selektiv begrenzt in den Randbereichen der Tropfen bzw. in den angrenzenden Bereichen zu den Tropfen anderer Tinten. Diese selektive Vernetzung kann entweder durch Bestandteile der Tinte oder durch gerichtete elektromagnetische Strahlung ausgelöst werden. Die Vernetzung kann biologischer, chemischer oder physikalischer Art sein. Die Tropfenbestandteile der speziellen Biotinten, die nicht im Randbereich liegen, werden ausgespült oder anders beseitigt, wodurch der notwendige Hohlraum für die entstehenden Kapillaren gebildet wird.

Dabei ins umliegende Gewebe ausgespülte, nicht vernetzte Kapillar-Zellen (z.B. Endothelzellen) werden z.B. zum Ausgangspunkt späterer neuer kapillarer Stukturen oder gehen bei der Gewebedifferenzierung unter.

Durch diesen Prozess wird es möglich, eine Druckauflösung zu erhalten, bei der nur einzelne Moleküle oder einzelne Zellen gezielt vernetzt werden. Die Eigenschaft, einzelne Zellen zu vernetzen, hatten bisher nur hochauflösende Stereolithographie-Verfahren. Die Tropfengrößen liegen dabei bevorzugt in Größen zwischen einem Femtoliter und 10 Mycrolitern.

Das so erzeugte Druckbild erhält eine neuartige, bisher unerreichte Qualität, bei der
- einerseits gleichzeitig verschiedene Tintentypen (z.B. Muskelzellen, Nervenzellen, Strukturtinten, Kapillartinten) in photorealistischer Weise gedruckt werden können und bei der
- andererseits durch die selektiv begrenzte Randvernetzung der Kapillartinten die Druckauflösung so hoch ist, dass einzelne Zell-Lagen (z.B. Endothelzellen) ausgebildet werden.

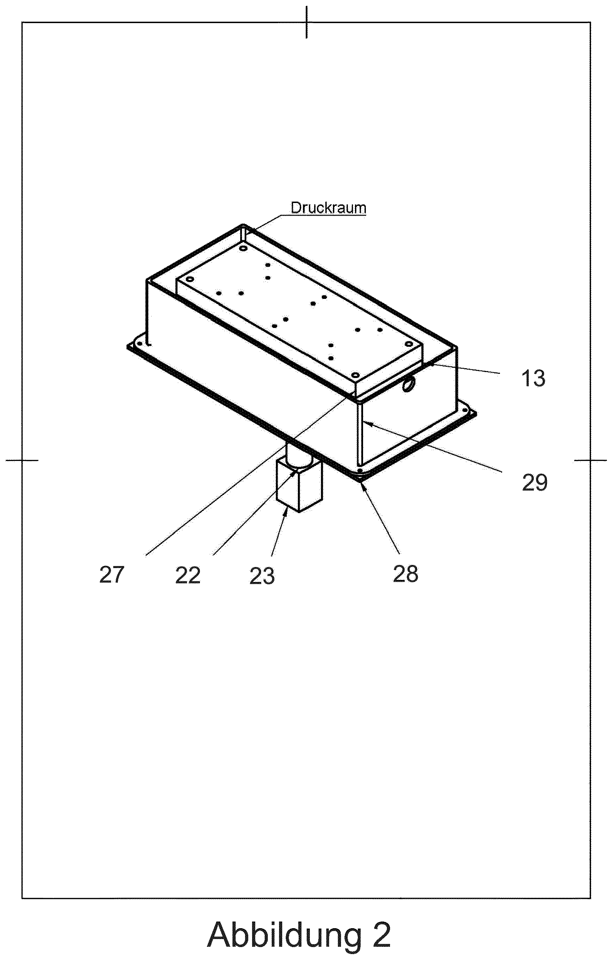

3) Durch die Ausbildung eines belastbaren Blutgefäßsystems bereits während des Drucks ist es möglich, schon während des Druckvorganges das entstehende Gewebe oder Organ ausreichend mit Nährstoffen zu versorgen. Die dazu erforderlichen Anschlüsse an Pumpensysteme werden zunächst durch den Druckertisch und die darauf befindliche Druckplatte (Abb. 2 - 7) bereitgestellt. Um noch feinere, für jeden Gewebeaufbau individuelle Anschlüsse für Gefäße und Kapillaren zu erzeugen, werden von diesen Anschlüssen aus individuelle Anschlüsse gedruckt. Dabei können in bevorzugten Ausführungsformen entweder Kapillar- und Gewebetinten oder zunächst eine Strukturtinte genutzt werden.

Falls dabei eine Strukturtinte genutzt wird, kann der Übergang zwischen Stukturtinte und Zelltinte über einen Gradienten erfolgen, wodurch das System stabilisiert wird. Bisher gibt es keine Droplet-Technologie, in der ein solches Versorgungssystem für gedruckte Zellen besteht.

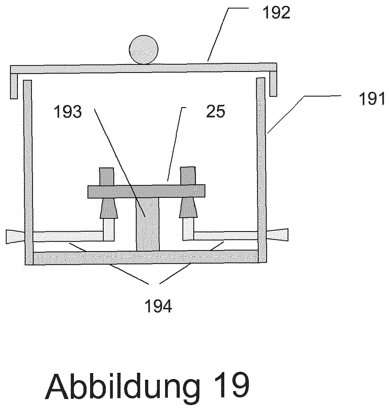

Nach erfolgtem Druck kann die herausnehmbare Druckfolie (auf der sich der fertige Druck befindet und die Zu- und Ablaufanschlüsse für Flüssigkeiten besitzt), in einem Inkubator weiter kultiviert werden (**Abb. 19**).

4) Es wird ein neuartiges - aber nicht beanspruchtes - komplexes Druckmuster offenbart, das die Ausbildung von Kapillaren zwischen arteriellen und venösen Strukturen ermöglicht, in denen die darin fließende Flüssigkeit (z.B. Medium oder Blut) vom arteriellen Bereich in den venösen Bereich strömt. Bisher sind solche Druckmuster nicht bekannt und könnten aufgrund des bisherigen Standes der Technik auch nicht umgesetzt werden.

Das Druckmuster ist deswegen einzigartig, weil in einem einzigen Druckvorgang geordnet verschiedene Zelltypen gedruckt werden und gleichzeitig geordnet Strukturen erzeugt werden können, die nur wenige Moleküle groß sind.

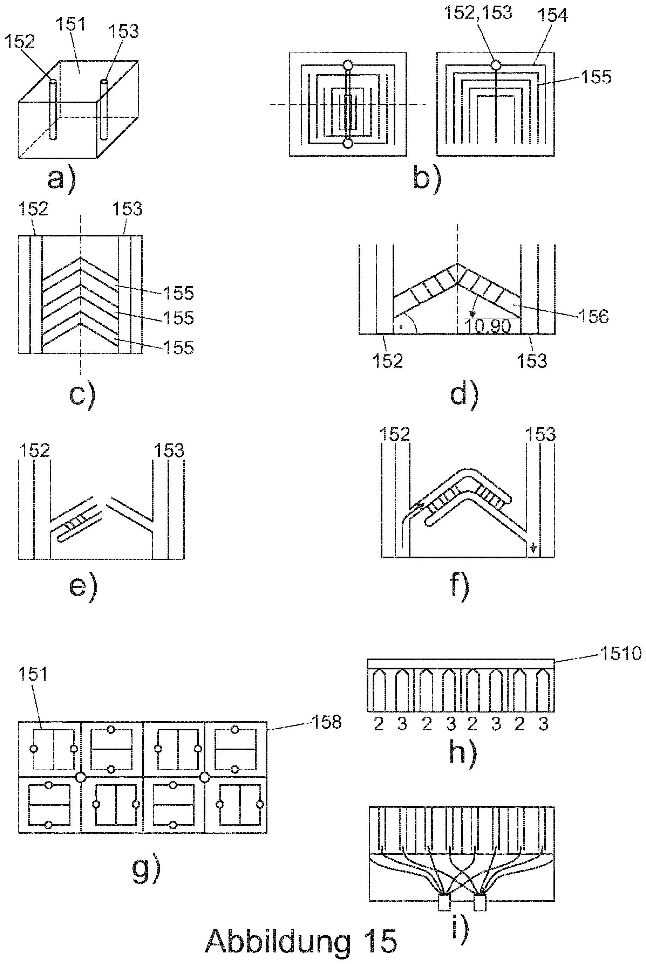

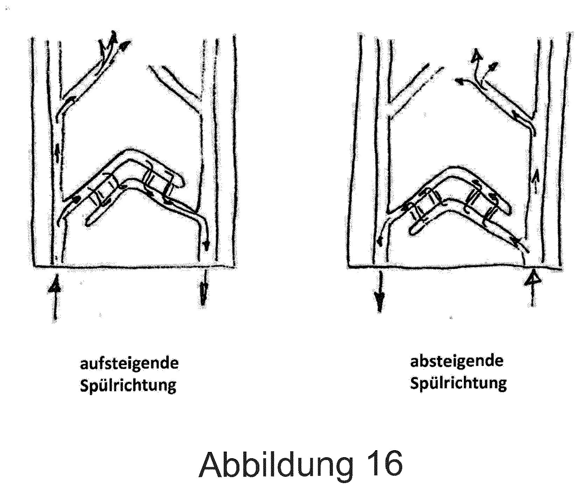

Dieses Druckmuster ist bevorzugt durch folgende Eigenschaften gekennzeichnet:
- Spezifischer Algorithmus zur Berechnung des Druckbildes in Bezug auf den verwendeten Zelltyp (Tintentyp) sowie in Bezug auf den Abstand und die Größe der Kapillaren und der arteriellen und venösen Strukturen. Dabei richtet sich der Algorithmus einerseits nach den Erfordernissen für eine optimale kapillare Versorgung des zu druckenden Moduls und andererseits nach den physiologischen und anatomischen Erfordernissen des jeweiligen Gewebaufbaus.
- Das aus unterschiedlichen Gewebetypen und Gefäßen bestehende Gewebe/Organ wird in einzelne Module unterteilt, die wiederum in Druckebenen (Slices) unterteilt werden. Die verschiedenen gedruckten Zelltypen auf einer Druckebne im Modul entsprechen dabei ihrer Gefäß- oder Gewebeposition innerhalb des jeweiligen Gewebes oder Organs.
- Es wird bevorzugt ein flächendeckendes Kapillarsystem mit einem Kapillar-Innendurchmesser von 5 Mycrometern und größer erzeugt. Die Kapillaren haben bevorzugt einen Abstand von ca. 50 Mycrometern zueinander auf jeder einzelnen Druckebene. Die darin fließende Flüssigkeit strömt nach abgeschlossenen Druck von arteriell nach venös.
- Die Summe der einzelnen Druckebenen führt zu einem Einzelmodul. Das Modul besitzt vorzugsweise ein aufsteigendes und ein absteigendes Gefäß, (**Abb. 15** **und** **16**), beide sind durch dazwischen liegende fächerförmige kleinere Kapillaren miteinander verbunden. Aus den aufsteigenden und absteigenden Gefäßen werden die Anschlüsse für die arterielle Zuführung und die venöse Abführung. Der dadurch entstehende Mikrokreislauf stellt die kleinste Versorgungseinheit im gedruckten Gewebe dar.
- Basismodule werden zusammengesetzt und verklebt zu größeren Geweben oder Organen. Die auf- und absteigenden Gefäße aus den Mikrokreisläufen werden dabei zu zu- und abführenden Hauptgefäßen vereint.

5) Bereitstellung eines neuartigen Druckertisches, der sich in Z-Richtung eines mediumgefüllten Druckraums bewegen kann und der Versorgungsanschlüsse hat, durch die das gedruckte Gewebe mit Medium regulierbar versorgt wird.
6) Bereitstellung einer Druckerkopfversorgungseinheit, in der regulierbar die Zelldichte und die Tintenkonzentration gemischt werden. Die gemischte Tinte für die Druckerköpfe wird nach Vorgaben des Druckeralgorithmus zusammengesetzt. Prinzipiell existieren im Verfahren Gewebetinten, Kapillartinten und Strukturtinten, wobei auch weitere Tintentypen vorkommen können.

### Gegenstand der Erfindung:

Es bedarf eines 3D-Druckverfahrens, das
- innerhalb eines Arbeitsschrittes verschiedene Zelltypen selektiv drucken kann und
- dass die hohe Druckauflösung von Stereolithographie-Verfahren erreicht.
- Zusätzlich soll das Verfahren eine Druckgeschwindigkeit besitzen, mit der der Druck von ganzen Organen real durchführbar ist.
- Außerdem sollte das neuartige Drucksystem bevorzugt bereits während des Drucks neu gebildetes Gewebe über die dabei erzeugten Kapillaren mit Medium oder anderen zur Ernährung der Zellen geeigneten Flüssigkeiten durchspülen (**Abb. 16**).
- Das Druckverfahren sollte bevorzugt auch ein neuartiges Druckmuster bereit stellen, mit dem es möglich ist, ein Kapillarsystem zwischen arteriellen und venösen Strukturen auszubilden, in dem die darin fließende Flüssigkeit (z.B. Medium oder Blut) von arteriell nach venös strömen kann.
- Das Drucksystem sollte insbesondere eine Vorrichtung besitzen, die für eine ausreichend hohe ZellKonzentration pro Volumeneinheit im Tintentropfen sorgt, ohne dass die Zellen dabei im Druckerkopf verklumpen.

Die Erfindung umfasst ein 3D-Druckverfahren gemäß Anspruch 1 sowie eine Steuereinheit gemäß Anspruch 10.

Das erfindungsgemäße 3D-Druckverfahren wird mit einem hochauflösenden Droplet-Drucker und einer Vorrichtung zum Applizieren von elektromagnetischen Wellen bevorzugt einem Laser, einer UV-Lampe oder einer Diode umgesetzt. Die dabei verwendeten Tinten sind niedrigviskose Tinten, die von den hochauflösenden Druckerköpfen photorealistischer Kunstdrucker verarbeitet werden können und die unmittelbar unter dem Einfluss elektromagnetischer Wellen vernetzen, bevorzugt innerhalb einer Sekunde. Die Tintentropfen können Volumina von 1 Femtoliter bis zu 10 Mycrolitern besitzen.

Das erfindungsgemäße 3D-Druckverfahren beinhaltet ebenfalls mindestens eine Kapillartinte, die in den Randbereichen der Tropfen bzw. in den angrenzenden Bereichen zu den Tropfen anderer Tinten selektiv vernetzt. Diese selektive Vernetzung erfolgt entweder hervorgerufen durch Tintenbestandteile (der Kapillartinte und/oder der angrenzenden Biotinten) oder durch gerichtete elektromagnetische Wellen, die einzelnen Zellen im Randbereich der Kapillartintentropfen vernetzen. Die nicht vernetzten Bereiche der Kapillartinten werden ausgespült oder auf andere Weise beseitigt und bilden den Hohlraum für das Kapillar- und Blutgefäßsystem.

Durch die selektiv auf den Randberiech der Kapillartintentropfen räumlich begrenzte Vernetzung können nicht nur einzelne Zellen, sondern sogar einzelne Moleküle zu einer Vernetzungsreaktion oder anderweitigen Schichtbildung aktiviert werden. Durch diesen Vorgang erreichte Druckauflösung ist noch höher als die von Stereolithographie-Verfahren, die bei ca. 20 nm liegt.

Beispielhafte Reaktionen im Randbereich der Kapillartintentropfen können sein:
- Thiol-en Reaktionen
- Schlüssel-Schloss-Reaktionen
- nucleophile Ringöffnungen
- Selfassembling von Molekülen und Partikeln
- selektive Faktoren an den Zellen der Kapillartinten und/oder Biotinten
- radikalische oder kationische Polymerisierungsreaktionen
- Antikörper-Reaktionen
- Reaktionen mit Klick-Chemie-Komponenten (z.B. Cycloaddition, andere)
- andere Vorgänge, in denen es möglich ist, dass zwei benachbarte Randbereiche eine räumlich begrenzte Reaktion oder Interaktion untereinander durchführen können oder in denen es zu einem "Self-assembling"-Prozess kommt

Da die für den jeweiligen Gewebedruck erforderlichen Tintenbestandteile im Tintentropfen bezüglich Zelldichte und Tintenkonzentration variieren können und vom Druckalgorithmus bestimmt werden, ist gemäß Anspruch 12 eine dem Druckerkopf vorgeschaltete Druckerkopfversorgungseinheit bereitzustellen (**Abb. 8**). In dieser werden die angezüchteten Zellen aus dem Zelltank über eine Pumpe in einen Zellkonzentrator gepumpt. Hier wird die Zelldichte aufkonzentriert und überschüssiges Medium abgesondert. Über einen Zellzähler wird eine definierte Zellkonzentration weitergeleitet zu einer Mischeinheit. In diese Mischeinheit wird ebenfalls aus einem Tank für Tintenkonzentrat ein bestimmtes Volumen eingeleitet. Die Mischeinheit mischt die Tinten nach Vorgaben des Druckalgorithmus für den erforderlichen Gewebetyp und leitet diese an die Druckerköpfe weiter. Die Anzahl der Druckerkopfversorgungseinheiten richtet sich nach der Anzahl der benötigten Tinten und kann variieren. Die Tinten könne jedoch auch ohne Vorgaben des Druckalgorithmus gemischt werden.

Um das gedruckte Gewebe am Leben zu erhalten, kann es z.B. erforderlich sein, dieses schon während des Druckvorgangs an einen Versorgungskreislauf anzuschließen. Außerdem muss sich das gedruckte Gewebe während des Drucks bevorzugt in einem mediumgefüllten Druckraum befinden, damit es nicht austrocknet. Herkömmliche Druckertische verfügen nicht über ein solches Versorgungssystem. Eine Aufgabe oder ein Aspekt der Erfindung war es deshalb, einen Druckertisch bereitzustellen, der während des Drucks das Gewebe mit Nährmedium durch Mediumzulauf und - ablauf versorgt (**Abb. 2**-7).

Die Flüssigkeit für Mediumzulauf und -ablauf wird z.B. von außen durch ein regulierbares Pumpsystem an den Drucker herangeführt. Die Laufrichtung der Pumpen ist dabei umkehrbar. Die Zuführung erfolgt insbesondere über ein Schlauchsystem, welches über Anschlussverbindungen (z.B. Luer) an den Druckertischfuß angeschlossen wird. Über die Zapfen des Druckertischfußes wird das Medium durch den Druckertisch in die Druckplatte geleitet. Zwischen dem Druckertischfuß und der Druckplatte befindet sich eine austauschbare Silikonmembran, die den mit Medium gefüllten Druckraum von der Bodenplatte trennt und gegen das Medium abdichtet (**Abb. 2-7**). Die Silikonmembran ist zur Dichtung notwendig, da die Bodenplatte des Druckraums vom Stift der Mycrometerschraube durchdrungen wird und den Druckertisch auf- und abfahren lässt.

Herkömmliche Dichtungssysteme wie beispielsweise Gleitringdichtungen sind erfahrungsgemäß potentielle Kontaminationsquellen. Die neuartige Lösung des Problems durch eine herausnehmbare Silikonmembran hat den Vorteil, dass sie leicht zu reinigen und zu sterilisieren ist. Außerdem kann man sie preiswert ersetzen. Durch ihre hohe Dehnbarkeit ist die Druckertischeinstellung frei wählbar. Die Silikonmembran fungiert gleichzeitig als Dichtung zwischen Bodenplatte und Druckraumwand.

Um den Drucktisch (innerhalb des Druckraums) problemlos wechseln zu können, besitzt der Drucktischfuß (außerhalb des Druckraums) insbesondere einen Magneten, der den Drucktisch anzieht. Durch die Magnetkraft werden ebenfalls die Anschlusszapfen des Druckertischfußes in die Dichtung der Gegenbohrung der Druckplatte gedrückt und abgedichtet. Diese bevorzugte erfindungsgemäße Lösung führt zu einer extrem einfachen Handhabung beim Wechseln des Drucktisches.

Das zu druckende Gewebe sollte, um es ausreichend versorgen zu können, von vielen Gefäßen durchdrungen sein (**Abb. 15**). Um das dazu erforderliche Druckmuster bereitstellen zu können, bedarf es insbesondere eines spezifischen Algorithmus in Bezug auf den verwendeten Zelltyp (Tintentyp) sowie in Bezug auf den Abstand und die Größe der Kapillaren und der arteriellen und venösen Strukturen. Dabei richtet sich der Algorithmus insbesondere einerseits nach den Erfordernissen für eine optimale kapillare Versorgung des zu druckenden Moduls und andererseits nach den physiologischen und anatomischen Erfordernissen des jeweiligen Gewebaufbaus. Das aus unterschiedlichen Gewebetypen und Gefäßen bestehende Gewebe/Organ wird insbesondere in einzelne Module unterteilt, die wiederum in Druckebenen (Slices) unterteilt werden. Die verschiedenen gedruckten Zelltypen auf einer Druckebene im Modul entsprechen dabei ihrer Gefäß- oder Gewebeposition innerhalb des jeweiligen Gewebes oder Organs. Es wird insbesondere ein flächendeckendes Kapillarsystem mit einem Kapillar-Innendurchmesser von ca. 10 Mycrometern und größer erzeugt. Die Kapillaren haben bevorzugt einen Abstand von ca. 50 Mycrometern zueinander auf jeder einzelnen Druckebene. Die darin fließende Flüssigkeit strömt nach abgeschlossenen Druck von arteriell nach venös (**Abb. 15**).

Die Summe der einzelnen Druckebenen führt zu einem Einzelmodul. Das Modul besitzt vorzugsweise ein aufsteigendes und ein absteigendes Gefäß, beide sind durch dazwischen liegende fächerförmige kleinere Kapillaren miteinander verbunden. Aus den aufsteigenden und absteigenden Gefäßen werden die Anschlüsse für die arterielle Zuführung und die venöse Abführung. Der dadurch entstehende Mikrokreislauf stellt die kleinste Versorgungseinheit im gedruckten Gewebe dar. Einzelmodule werden zusammengesetzt und verklebt zu größeren Zielstrukturen.

Die für das Gefäßsystem bevorzugten erforderlichen Anschlussöffnungen, auf die die Gefäße gedruckt werden, befinden sich auf einer Druckplatte, die auf dem Drucktisch befestigt wird (**Abb. 2** - 7). Die Funktion dieser Druckplatte ist die Verteilung des Mediums aus den beiden Hauptanschlüssen des Drucktisches in die individuell erforderlichen Nebenanschlüsse. Da jedes Gewebe anatomisch anders aufgebaut ist, sollte die Platte für jeden Druckvorgang individuell erstellt werden. Jede Druckplatte wird im Vorfeld über einen 3D-Druck (z.B. FDM, SLS, BJ, andere) angefertigt. Die Dateien zum Druck stammen ebenfalls aus dem Systemalgorithmus zur Organisation des Druckers. Die Druckplatte entspricht einem individuell erstellten Arbeitsmittel für den Druck und ist bevorzugt aus einem Polymer gefertigt.

Oberhalb der Druckplatte ist eine Druckfolie bevorzugt aus bioabbaubarem bzw. resorbierbarem Polymer aufgebracht, die die gleichen Anschlüsse wie die Druckplatte besitzt. Die Druckfolie dient dem leichten und zerstörungsfreien Ablösen des Gewebes von der Druckplatte. Durch die biologische Abbaubarkeit des Polymers wird die Druckfolie in kurzer Zeit im Körper abgebaut (**Abb. 2-7**).

Um das gedruckte Gewebe problemlos in einem handelsüblichen Brutschrank reifen zu lassen, wird der Drucktisch mit Druckplatte (fakultativ nur die Druckplatte) in ein Inkubatorgefäß (**Abb. 19**) überführt, das im Brutschrank gelagert wird. Dieses Inkubatorgefäß besteht insbesondere aus einer mediumgefüllten Wanne mit einem aufzulegenden Deckel. Auf dem Boden der Wanne ist ein Fuß fest installiert, auf dem ein baugleicher Druckertischfuß wechselbar angebracht ist. Die zu- und abführenden Mediumschläuche zum Druckertischfuß werden über Öffnungen im Inkubatorgefäß herangeführt. Auf die Zapfen des Druckertischfußes (ohne Silikonmembran) wird mittels Magnetkraft der Druckertisch samt Druckplatte, Druckfolie und gedrucktem Gewebe aufgesetzt (**Abb. 19**).

### Weitere Ausführungsformen:

In einer bevorzugten Ausführungsform der Erfindung liegt die Tropfengröße zwischen einem Femtoliter und 10 Mykrolitern. Vorteilhafterweise erlaubt diese Tropfengröße die Lösung der erfinderischen Aufgabe überraschend gut in einer Thiol-en Reaktion. Thiolmodifizierten Moleküle im Inneren des Tropfens vernetzen unter diesen Bedingungen nicht und werden ausgespült.

In einer weiteren bevorzugten Ausführungsform besitzen die Kapillartinten thiolmodifizierte Moleküle, die sie umgebenden Biotinten besitzen allylmodifizierte Moleküle und einen Photoinitiator zur Auslösung der Vernetzungsreaktion. Diese Vernetzungsreaktion findet innerhalb der Biotinten zwischen den allylmodifizierten Molekülen statt. Die thiolmodifizierten Moleküle der Kapillartinten vernetzen nur selektiv im Randbereich mit den allylmodifizierten Molekülen der Biotinten.

In einer weiteren bevorzugten Ausführungsform führt eine Kupfer(I)-katalysierte 1,3-dipolare Cycloaddition von Aziden und Alkinen zur Vernetzungsreaktion im Randbereich der Kapillartinten, wobei azidmodifizierte und alkinmodifizierte Moleküle nur im Grenzbereich der Kapillartintentropfen zu Biotinten aufeinander treffen.

In einer weiteren bevorzugten Ausführungsform enthält die Kapillartinte epoxidgruppenmodifizierte Moleküle, die eine nukleophile Ringöffnung mit OH-Gruppen oder NH₂-Gruppen von Molekülen aus den Biotinten hervorrufen können oder die durch Komponenten aus den Biotinten eine säurekatalysierte oder basenkatalysierte Ringöffnung durchlaufen.

In einer weiteren bevorzugten Ausführungsform enthalten Kapillartinten und Biotinten Moleküle, die im Grenbereich self-assembling Strukturen ausbilden, beispielsweise über Ionen, Aptamere oder andere zu self-assembling neigenden Strukturen.

In einer weiteren bevorzugten Ausführungsform der Erfindung ist vorgesehen, dass die Zellen einen selektiven Faktor enthalten, durch den sie mittels gerichteter Bewegung der elektromagnetischen Wellen innerhalb eines Tropfens selektiv vernetzt werden.

Die neuartigen niedrigviskosen, durch elektromagnetische Wellen vernetzbaren Biotinten und Kapillartinten setzen sich zusammen aus a) dem jeweiligen Zelltyp (fakultativ mit für den Zelltyp selektivem Faktor) und Vernetzungsmolekülen.). Zusätzlich können auch noch Wachstumsfaktoren, Signalmoleküle, Partikel für den Aufbau einer Reizleitung oder andere Moleküle Bestandteile der Tinte sein.

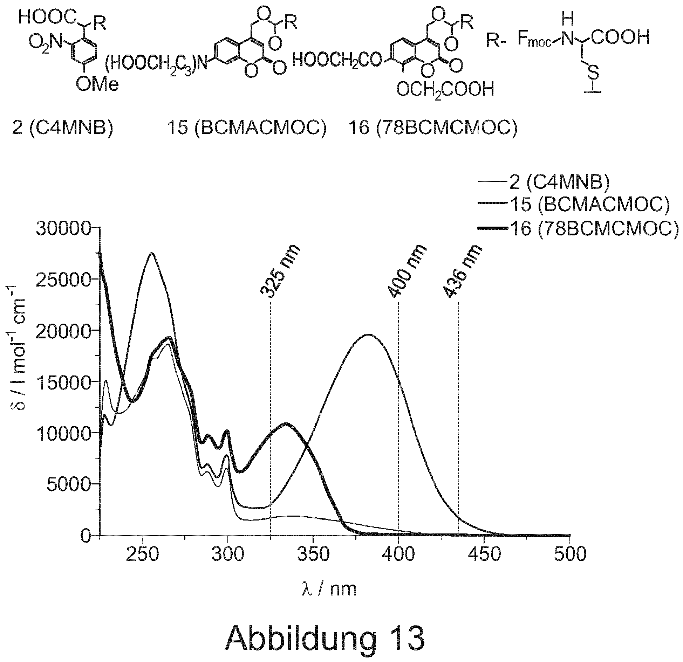

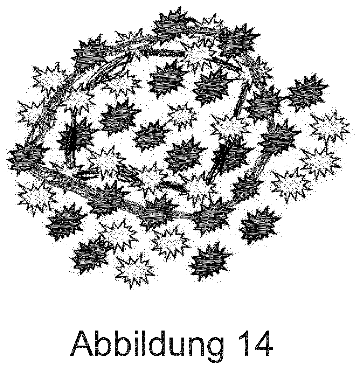

Durch eine selektiv wirkende Energiequelle kann aus einem Tropfen mit einer Mischung verschiedener Zellen eine Vernetzungs- oder Polymerisationsreaktion ausgelöst werden, in der nur der jeweils beabsichtigte Zelltyp getroffen wird. Bevorzugt können die nicht vernetzten Zellen entfernt werden. Beispielsweise kann ein kleiner Tropfen von nur 5 Pikolitern (ca.20 - 200 Zellen) aus einem Gemisch von vaskularen Endothelzellen und Myoblasten bestehen. Aus diesem Gemisch heraus wird in einer bevorzugten Ausführungsform anschließend mit einem nanometergenauen Laserstrahl der Ring einer feinen Kapillare geformt. Durch einen spezifischen Energieeintrag eines Lasers werden im Gemisch bevorzugt nur die Endothelzellen vernetzt, die sich entlang der Innenseite des Ringes befinden. Mit einem anderen spezifischen Energieeintrag eines Lasers werden in dieser bevorzugten Ausführungsform auf der äußeren Seite des Ringes nur die angrenzenden Myoblasten vernetzt (Abb. 13 und 14)

In einer weiteren bevorzugten Ausführungsform ist vorgesehen, dass die nicht vernetzten Zellen im Inneren der vaskularen Struktur mittels Laser entfernt werden.

Bevorzugt werden auch Biotinten bestehend aus einem insbesondere homogenen Zelltyp vernetzt, um beispielsweise größere homogene Gewebebereiche zu drucken.

In einer bevorzugten Ausführungsform werden die zu vernetzenden Zellen in ein sich bildendes Netzwerk eingeschlossen.

In einer weiteren Ausführungsform werden die Zellen selbst untereinander oder mit einem Netzwerk vernetzt. Dazu besitzen sie ein Vernetzungsmolekül, das in der Zellmembran verankert ist.

Durch den vergleichsweise schnellen Druckprozess ist es überraschend möglich, die gedruckten Gewebe relativ zeitnah in einen Inkubator zu überführen, in dem durch Umgebungsfaktoren (z.B. chemische, molekularbiologische, oder durch Strömungsbelastung) eine weitere Reifung ausgelöst wird.

Bevorzugt werden weitere vernetzbare Tinten an bestimmten Positionen verdruckt, die Stützfunktionen für das Gewebe tragen, die Faktoren tragen, die für die Physiologie der Zelle wichtig sind, oder die Wachstumsfaktoren, Marker, Signalmoleküle, Rezeptoren oder weitere Bindungsstellen (Schlüssel-Schloss-Prinzip) tragen.

Der Druckerkopf besitzt ähnlich dem Tintenstrahldrucker für photorealistische Kunstdrucke bevorzugt eine größere Anzahl von Düsen, die auf die jeweilige Rheologie der Tinte angepasst sind. Der Drucker besitzt bevorzugt auch eine oder mehrere Quellen für ungerichtete oder gerichtete elektromagnetische Wellen, durch die die Vernetzungsreaktionen ausgelöst werden.

In einer bevorzugten Ausführungsform wird ein Druckerkopf für photorealistische Kunstdrucke genutzt, der zusätzlich mit einer oder mehreren Quellen für elektromagnetische Wellen modifiziert wurde.

In einer weiteren Ausführungsform wird ein Druckerkopf benutzt, der aus Modulen zusammengesetzt ist, wobei jedes Modul einem Druckerkopf für photorealistische Kunstdrucke mit einer oder mehreren Quellen für elektromagnetische Wellen entspricht.

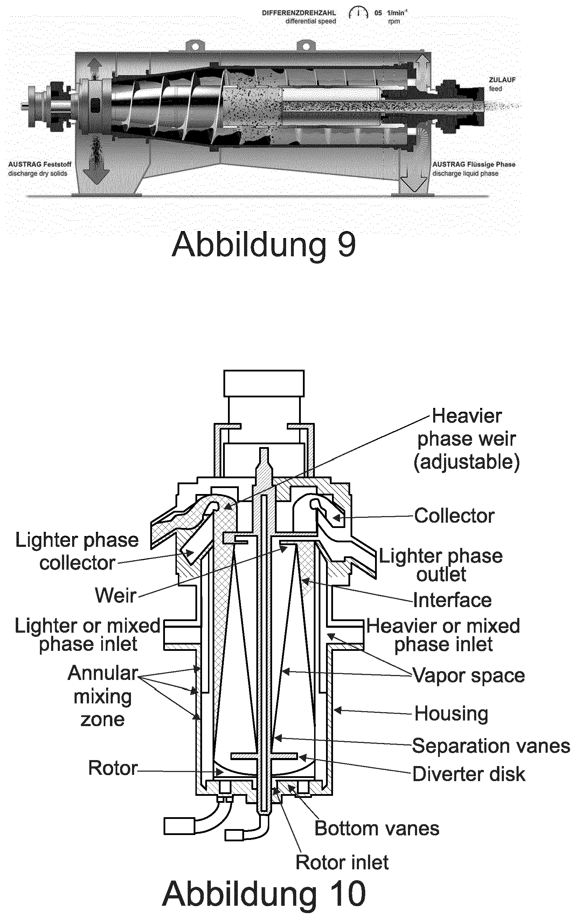

In einem weiteren Aspekt der Erfindung betrifft diese einen neuartigen Aufbau eines Druckerkopfs (siehe Abb. 9 bis Abb.12) sowie einen neuartigen Druckvorgang, in dem verschiedene Energiequellen selektiv zur Auslösung von verschiedenen Reaktionen genutzt werden (Abb. 9-12).

Abb. 13 und Abb. 14 zeigt schematisiert, wie über verschiedene Photoinitiatoren mit Hilfe von Licht einer spezifischen Wellenlänge bestimmte Zellen miteinander vernetzt werden können. Als Energiequelle können Laser, Dioden oder andere Energiequellen dienen (siehe Abschnitt Beispiele).

In einer bevorzugten Ausführungsform wird das gedruckte Gewebes während des Druckvorgangs ernährt. Die an der Bodenplatte vorhandenen Anschlüsse für das Nährmedium werden individuell auf das zu druckende Gewebe durch Strukturtinten erweitert. Ausgehend von den Anschlüssen an der Bodenplatte werden mit einer Strukturtinte feinere Anschlüsse mit kleineren Durchmessern gedruckt.

In einer weiteren Ausführungsform ist der Druckerkopf mit einer Bubble-Technologie, einer Piezo-Technologie oder anderen Druckerkopftechnologien ausgestattet.

Die Quelle für elektromagnetische Wellen ist in einer Ausführungsform eine ungerichtete Quelle, beispielsweise eine Diode, ein Laser, eine UV-Lampe oder eine andere Quelle.

Die Quelle für elektromagnetischen Wellen ist in einer weiteren Ausführungsform eine um 180° schwenkbare Linse. Die Energiequelle ist steuerbar und beweglich, so dass der Laserstrahl jede vorprogrammierte Form abbilden kann. Das Licht wird in einer weiteren Ausführungsform in einer externen Lichtquelle generiert und über Lichtleiterkabel zum Austrittsort transportiert.

In einer Ausführungsform ist die Wellenlänge der elektromagnetischen Wellen im UV-Bereich, im sichtbaren Bereich oder im IR-Spektrum.

In einer bevorzugten Ausführungsform ist zur Beseitigung des ausgeschwemmten nicht vernetzten Tintenmaterials eine Absaugvorrichtung oder eine Druckluftvorrichtung vorhanden.

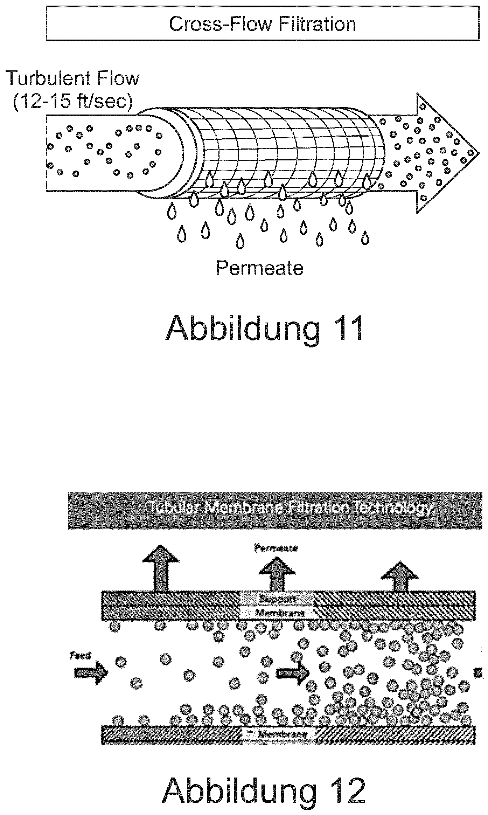

In einer Ausführungsform ist das Vakuum steuerbar und wird nur bei gesenkter Absaugvorrichtung aktiviert. Der Abstand zwischen Absaugvorrichtung und dem zu druckenden Objekt wird durch einen Laser kontrolliert (Abb. 11 und Abb. 12).

In einer weiteren Ausführungsform bedarf es zur Beseitigung des ausgespülten Tinten- bzw. Zellmaterials keiner zusätzlichen Vorrichtung, weil die ausgespülten Zellen in den umliegenden Geweben Ausgangspunkte für neue Kapillaren bilden oder bei der Differenzierung des umliegenden Gewebes mit eingebaut werden oder untergehen.

In einer weiteren Ausführungsform wird das nicht vernetzte Tintenmaterial durch einen gerichteten Laserstrahl zerstört.

In einer weiteren Ausführungsform werden die nicht vernetzten Zellen, Zelltrümmer oder Tinten durch eine Vorrichtung ausgespült und entweichen in den Reaktionsraum.

In einer Ausführungsform wird über Begasungsdüsen eine Gasatmosphäre oberhalb der Druckebene erzeugt (z.B. Inertgas, CO2, usw.)

In einer Ausführungsform ist die Wellenlänge und die Strahlbreite der Quelle für elektromagnetische Wellen modulierbar. Der Drucker besitzt eine oder mehrere modulierbare Quellen für die gezielt gerichteten elektromagnetischen Strahlen.

In einer weiteren Ausführungsform wird bevorzugt ein Femtosecond Laser eingesetzt.

In einer Ausführungsform wird während des Drucks über das Nährmedium der Gasgehalt (z.B. CO_{2/}O₂), der pH-Wert und andere wichtige Zellkultur-Parameter geregelt. Zusätzlich wird der Gasaustausch auch über den Gasgehalt im Reaktionsraum reguliert.

In einer Ausführungsform wird das Verfahren als "Bottom Up" oder "up to Bottom" durchgeführt.

In einer Ausführungsform wird das Reaktionsgefäß des Druckers fakultativ als Einweg-Einsatz in den Drucker eingesetzt und kann nach dem Druck entnommen werden. Nach dem Druck ist das Gewebe oder Organ in einem Zustand, in dem es in Kontakt mit dem Patienten gebracht oder in die Zellkultur überführt werden kann. Das Reaktionsgefäß besitzt Anschlüsse für den Zu- und Ablauf von Nährmedium oder Blut zur Ernährung des Gewebes.

In einer Ausführungsform wird das Reaktionsgefäß gekühlt.

In einer - nicht beanspruchten - Anwendungsform im Patienten wird das Reaktionsgefäß außerhalb des Körpers mit dessen Blutkreislauf verbunden. Durch die bereitgestellten patiententypischen Wachstums- und Differenzierungsfaktoren sowie durch den Blutdruck innerhalb des sich noch differenzierenden vaskulären Systems kommt es zur weiteren Reifung. Während dieser Phase werden fakultativ Proben aus dem System entnommen, um den Reifegrad (Differenzierungsgrad) des Gewebes oder Organs zu überwachen und den optimalen Zeitpunkt für eine Implantation zu ermitteln. Für diese Probennahmen besitzt das Reaktionsgefäß weitere Anschlüsse.

Im Zusammenhang mit dem erfindungsgemäßen Verfahren ist es vorteilhaft, wenn bestimmte Zelltypen vor dem 3D-Druck mit Molekülen spezifisch hybridisiert werden, um die Zellen in einem Gemisch selektiv ansprechbar zu machen. Die Verbindung besteht hierbei aus einem selektiven Faktor und einem Ankermolekül. Der selektive Faktor soll bevorzugt die Quervernetzung auslösen. Das Ankermolekül dient bevorzugt zum Andocken an die Zellmembran. Der selektive Faktor kann auch direkt ohne Ankermolekül an der Zelloberfläche binden, falls das seine chemischen und physikalischen Eigenschaften zulassen. Ein räumlich gesteuerter, in seiner Wellenlänge und seiner Frequenz speziell gerichteter Energiestrahl aus elektromagnetischen Wellen trifft auf einen selektiven Faktor eines spezifischen Zelltyps und aktiviert diesen beispielsweise über die Abspaltung einer Schutzgruppe, die Aktivierung eines Photoinitiators, die Aktivierung eines Reaktionspartners oder die Aktivierung einer Schlüssel-Schloss-Komponente. Andere Zelltypen werden von dieser Vernetzungsreaktion nicht betroffen. (Abb. 13 und 14, siehe Abschnitt Beispiele).

In einer Ausführungsform besitzt der selektive Faktor als Ankermolekül ein lipophiles Molekül, beispielweise Perfluorcarbone oder Lipide. In einer anderen Ausführungsform besitzt der selektive Faktor als Ankermolekül kationische Molekülbereiche oder Molekülbereiche mit positiven Partialladungen. In einer weiteren Ausführungsfom besitzt der selektive Faktor als Ankermolekül Peptidverbindungen z.B. zellpenetrierende Peptide oder Polynukleotide.

Im Zusammenhang mit der erfindungsgemäßen Lehre werden neuartige Biotinten eingesetzt:
Im Gegensatz zu den bisher genutzten höherviskosen Biotinten herkömmlicher Dropletverfahren mit den darin enthaltenen langkettigen Makromolekülen sind die neuartigen Biotinten des hier offenbarten Verfahrens sehr niedrigviskos und enthalten Photoinitiatoren. Sie enthalten sehr kurzkettige kleinere Vernetzungsmoleküle, die unmittelbar nach dem Kontakt mit elektromagnetischen Wellen vernetzen. In der bevorzugten Ausführungsform besitzen diese Moleküle Allylmodifizierungen oder Thiolmodifizierungen.

In einer weiteren Ausführungsform wird der Druckraum begast.

In einer weiteren Ausführungsform werden die Kapillaren nicht in einem Winkel von 10-90 Grad zur Druckerplatte sondern parallel (0 -10 Grad) dazu gedruckt.

In einer weiteren Ausführungsform werden die Kapillaren bereits vor dem Ringschluss gespült.

### Allgemeiner Ablauf eines Drucks - theoretische Grundlagen und bevorzugte Ausführungen:

Es folgt eine Offenbarung von einem Verfahren zum 3D-Druck von Organen und Geweben. Größere Druckbereiche werden in Einzelmodule aufgegliedert, unabhängig voneinander gedruckt (auf dem gleichen oder auf verschiedenen Druckern) und anschließend zum Zielorgan oder -gewebe zusammengesetzt und verklebt (z.B. mit Fibrinkleber). Zu- und abführende Gefäße der Einzelmodule werden dabei zu zu- oder abführenden Hauptgefäßen zusammengeschlossen. Das Einzelmodul ist ein bevorzugt kubisches Gebilde mit definierter Kantenlänge.

Ein Gewebe oder ein Organ sind zwar vom Aufbau gleich aber nicht identisch. Jedes Individuum unterscheidet sich, auch bei gleicher Spezies, geringfügig von einem anderen. Das äußert sich nicht nur in der Größe, sondern auch in der individuellen Entwicklung. Die Muskeln eines Sportlers zum Beispiel sind aufgrund des höheren Sauerstoffbedarfs wesentlich stärker kapillarisiert als bei einem Nichtsportler. Der Körper passt sich den Umweltbedingungen an und ist daher niemals identisch und erlebt einen ständigen Auf- und Abbau.

Entscheidend ist daher die Funktion des Gewebes und nicht die exakte anatomische Wiedergabe des Gewebes des Patienten. Daher wird hier ein Modulsystem offenbart, welches die Funktion des Gewebes ermöglicht aber auch soweit vereinfacht und systematisiert wurde, dass der 3D- Druck des Gewebes ökonomisch durchgeführt werden kann.

Der Grundbaustein des Gewebes oder des Organs ist das kubische Einzelmodul (151) (**Abb.15-18**) von definierter Seitenlänge. Zur Versorgung des Gewebes, welches aus ein bis mehreren Zellarten bestehen kann und daher auch ein funktionelles Gewebe wie ein Organ bilden kann, wird an der einen Seite von einem aufsteigenden Gefäß (152), welches später die Arterie bildet, versorgt. Das an der gegenüberliegenden Seite befindliche absteigende Gefäß (153) bildet die Vene. Von den Gefäßen gehen jeweils fächerförmig kleinere Gefäße (154) ab, die sich bis zur Höhe des gegenüberliegenden Gefäßes auf der anderen Seite erstrecken und parallel zueinander verlaufen (Abb. 15a). Die kleineren Gefäße (154) sind durch Brückengefäße (156) miteinander verbunden, welche später die Kapillaren bilden und den "Blutkreislauf" schließen und einen Mikrokreislauf (**Abb. 15-18****)** bilden.

Der Mikrokreislauf (**Abb. 15**) stellt die kleinste Versorgungseinheit in dem gedruckten Gewebe dar. Hierbei wird über das aufsteigende Gefäß (Arterie) (152), das Medium in ein kleines Gefäß (154) geleitet, welches zum aufsteigenden Ast gehört. Von dem kleineren Gefäß strömt das Medium über die Brückengefäße (156) in ein darunterliegendes kleineres Gefäß (154), welches zum absteigenden Ast gehört und im absteigenden Gefäß (153) mündet.

Da beim Drucken der Gefäße mit der Kapillartinte viele Zellen pro Tropfen verdruckt werden und nur die Zellen an den Randbereichen in der Lage sind sich mit dem umliegenden Gewebe zu vernetzen, müssen die überschüssigen Zellen ausgespült werden. Dazu und zur Versorgung der gedruckten Zellen wird nach jeder Fertigstellung eines Mikrokreislaufs, dieser mit Medium für eine kurze Zeit durchspült. Bei jedem Spülvorgang werden die unterhalb des letzten Mikrokreislauf liegenden Mikrokreisläufe mit gespült und die Zellen versorgt. Der Fluss des Mediums spült die unvernetzten Zellen aus. Horizontal verlaufende Gefäße sind dafür nicht optimal, weshalb die gedruckten Gefäße alle in einem Winkel von 10 bis 90 Grad zur Druckerplatte verlaufen.

Die auf- und absteigenden Gefäße (**Abb. 15****,****16**) (152 und 153), steigen in einem Winkel von 90° an. Die Gefäßfächer (155) können in einem Winkel von 10 - 90° ansteigen. Gedruckt wird bis zur Mitte des Einzelmoduls aufsteigend, danach absteigend, sodass die kleineren Gefäße immer aufsteigend zur Druckerplatte verlaufen. So können bei den unfertig gedruckten Gefäßen die überschüssigen Zellen gut ausgespült werden. Wenn ein Mikrokreislauf geschlossen ist, kann das Medium über das absteigende Gefäß ablaufen (**Abb.15** **und** **16**).

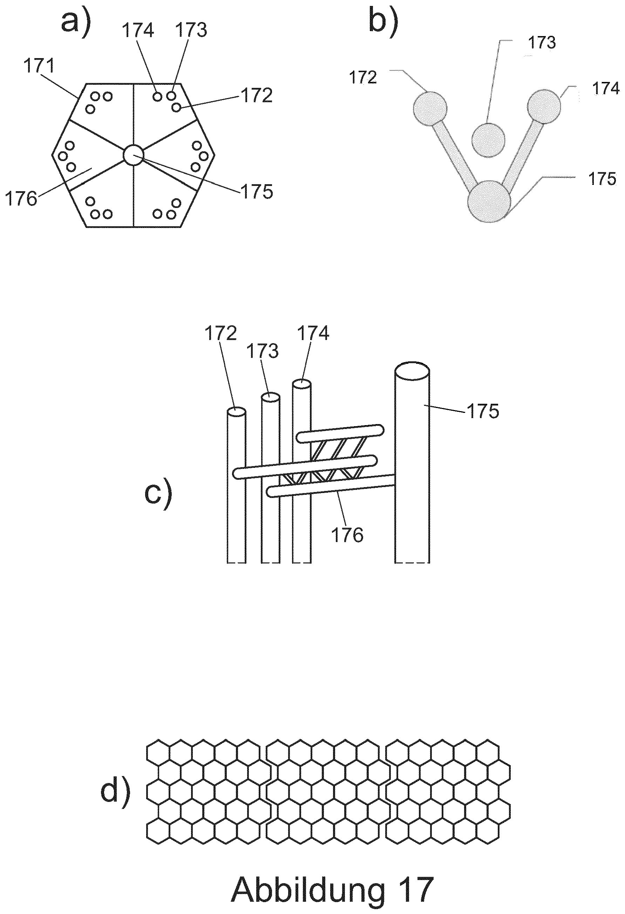

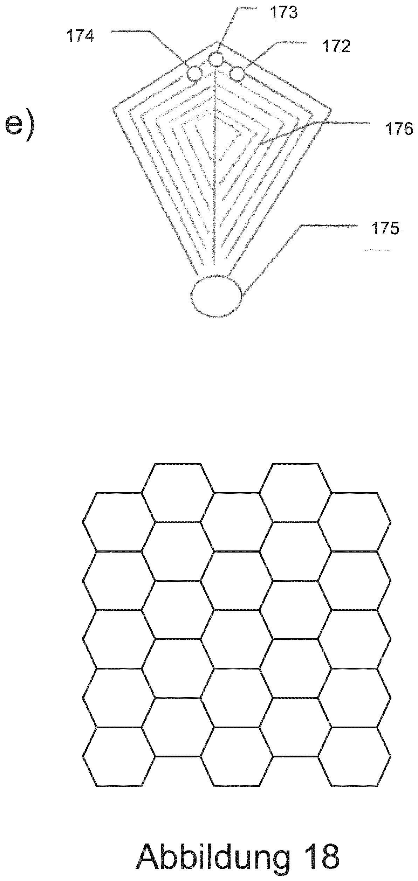

Die Gefäßfächer (**Abb. 15** **und** **16**) werden in einem definierten Abstand gedruckt, wobei der Gefäßfächer des auf - und absteigenden Gefäßes abwechselnd untereinander verlaufen. Jeweils ein Gefäßfächer des aufsteigenden Gefäßes (152) und des absteigenden Gefäßes (153) werden durch Brückengefäße (156) miteinander verbunden und bilden den Mikrokreislauf (**Abb. 15** **und** **16**) Dieser Vorgang wird so lange wiederholt, bis das Einzelmodul gefüllt ist. Zum Drucken eines Gewebes oder eines Organs, wird ein Gewebemodul (**Abb. 17**) (158) gedruckt welches aus mehreren Einzelmodulen besteht. Die Einzelmodule werden auf der Druckerplatte, in ihrer Ausrichtung versetzt (Abb. 15g), bündig nebeneinander gedruckt, sodass sich ein Gewebemodul von definierter Seitenlänge und Dicke ergibt. Die Organmodule können zeitlich parallel auf verschiedenen Druckern gedruckt werden und mittels eines Klebers (z.B. Fibrinkleber) zu größeren Gewebe- oder Organeinheiten verbunden werden (Abb. 15e).

Um die Gewebemodule an den Kreislauf des Körpers anschließen zu können, muss bevorzugt ein Gewebeabschlussmodul (158) (**Abb.15** **und** **17**) ) und ein Gewebeanschlussmodul (**Abb.15** **und** **17**) gedruckt werden.

Das Gewebeabschlussmodul (158) besteht aus einem Gewebemodul, bei dem sich in den Einzelmodulen die auf- und absteigenden Gefäße nach oben verjüngen und so verschließen und mit mehreren Zelllagen (1510) abgeschlossen werden.

Das Anschlussmodul muss bevorzugt die einzelnen Gefäße der der Einzelmodule zu größeren Gefäßen zusammenführen und dabei die anatomischen und chirurgischen Vorgaben berücksichtigen. Dabei verlaufen die zu - und abführenden Gefäße auf unterschiedlichen Ebenen zu den Anschlussgefäßen hin. Die aufsteigenden Gefäße und die absteigenden Gefäße sind wieder durch Brückengefäße zur Versorgung des Gewebes miteinander verbunden (**Abb.15****,****17**).

### Allgemeine Beschreibung des Druckvorgangs (Abb. 1-12):

Das zu druckende Gewebe wird um einen ökonomischen Druckvorgang zu erreichen bevorzugt in einzelne Gewebemodule unterteilt, die sich wiederum aus Einzelmodulen zusammensetzen. Das aus den Einzelmodulen bestehende Gewebemodul, sowie die Einzelmodule bilden einen Querschnitt aus einer definierten Ebene des gesamten Gewebes oder Organs mit seinen unterschiedlichen Zelltypen und Gefäßen. Diese Unterteilung erfolgt mit Hilfe eines spezifischen Algorithmus. Durch das Drucken der einzelnen Ebenen (Gewebeschnitte), entsteht wieder ein dreidimensionales Gewebe.

Entsprechend der Vorgaben des Gewebemoduls, wird die Druckplatte mit den Versorgungsöffnungen aus einem Polymer gedruckt.

Ebenso wird die Druckfolie aus einem bioabbaubaren Polymer geduckt und zusammen mit der Druckplatte auf dem Druckertisch befestigt und in den Druckraum eingesetzt.

Die Tanks für Kapillartinten, Gewebetinten und Strukturtinten werden befüllt und angeschlossen.

Die benötigten Zelltypen werden in einem externen, dafür ausgestatteten und zertifizierten Labor vermehrt und für den Druckvorgang vorkonzentriert und in den Tanks zum Drucker transportiert.

Die Behälter für den Medium Zu - und Ablauf werden befüllt und die zu- und abführenden Schläuche an die entsprechenden Anschlüsse am Druckertischfuß angeschlossen. Versorgt wird der Zu- und Ablauf über ein Pumpensystem, welches einen Vor- und Rücklauf besitzt. Der Druckerraum wird ebenfalls mit Medium gefüllt, welches über einen Zu- und Ablaufsystem konstant gehalten werden kann. Der Druckvorgang kann nun gestartet werden, die Druckplatte fährt in ihre Startposition.

Zuerst werden mit der Strukturtinte die Gefäßanschlüsse auf die Druckfolie auf der Druckplatte gedruckt. Danach druckt der Drucker Ebene für Ebene und bildet dabei Schicht für Schicht die einzelnen Gewebebereiche und Gefäße aus und sinkt dabei in das Medium des Druckerraums ab, bis das Gewebemodul fertig ist. Der Druckvorgang ist dabei so programmiert, dass in immer wiederkehrenden Zyklen nach dem Erreichen einer bestimmten Höhe des Gewebes ein kurzer Spülvorgang eingeleitet wird, um die überschüssigen und unvernetzten Zellen auszuspülen. Dieser Spülprozess kann in verschiedenen Ausführungsformen vorliegen. Bevorzugt wird nach einer definierten Anzahl an Druckvorgängen einmal der aufsteigende Ast gespült, dann nach einer weiteren definierten Anzahl an Druckvorgängen der absteigende Ast. Immer alternierend, bis das Gewebemodul fertig gedruckt ist. Durch den alternierenden Spülvorgang können die zu - und ablaufenden Gefäße gespült werden, ohne die Gefäße zu überlasten.

Wenn das Einzelmodul fertig gedruckt wurde, kann es mit Hilfe der Druckfolie problemlos vom Drucktisch gelöst werden. Die gedruckten Einzelmodule werden mit einem Kleber (z.B. Fibrinkleber) zu einem vollständigen Gewebe oder Organ zusammengesetzt. Die zu- und abführenden Gefäße der Einzelmodule werden zu zu- und abführenden größeren Gefäßen vereinigt.

Über den im Gewebe gedruckten arteriellen und venösen Kreislauf kann das Gewebe weiter mit Medium versorgt werden und in einem Inkubator noch mehrere Tage reifen, bevor es transplantiert wird.

### Einzelmodule am Beispiel der Leber (Abb. 15, 16, 17, 18):

Die Leber besteht aus ca. 1 - 1,5 Millionen Leberläppchen mit einem Durchmesser von 1-2 mm. In die Leber enden zwei Gefäße, die Leberarterie zur Versorgung des Gewebes mit Sauerstoff und Nährstoffen und die Pfortader, die das Blut aus Magen und Darm mit den aufgenommenen Nährstoffen und Giftstoffen transportiert. Aus der Leber heraus gehen die Venen die sich zur Hohlvene vereinen und der Gallengang.

Das Einzelmodul wird hier aus den einzelnen hexagonalen Leberläppchen (171) (Abb.17a) mit einem Durchmesser von 2 mm gebildet. Die Leberläppchen sind nebeneinander in 5 Reihen angeordnet und jeweils 5 Reihen hoch (Abb. 17d). Durch die Anordnung der Leberläppchen in den Reihen, das jede zweite Reihe um ein halbes Leberläppchen versetzt ist, lassen sie sich bei der Bildung von Organmodulen verzahnen.

Die einzelnen hexagonalen Leberläppchen haben in jeder Ecke 3 Gefäße (Arterie (172), Pfortader (174), Gallengang) und in der Mitte die Vene (175). Alle 4 Gefäße bilden wieder einen Mikrokreislauf (Abb.17b).

Das Leberläppchen ist in jeweils zu den einzelnen Eckgefäßen in 6 Segmente (176) unterteilt, die von den Mikrokreisläufen durchströmt werden und einen Gefäßfächer bilden. Die beiden zuführenden Gefäße, die Arterie und die Pfortader laufen im Mikrokreislauf parallel zueinander zur Vene hin. Ihnen entgegen kommt aus der Mitte die Vene, sie verläuft unterhalb der zuführenden Gefäße, so bilden die drei Gefäße ein Dreieck. Die Arterie und die Pfortader sind wieder mit Gefäßbrücken mit der Vene verbunden und bilden einen Mikrokreislauf. Der Gallengang befindet sich in der Mitte des Gefäßdreiecks.

### Druckvorgang eines Lebermoduls (Abb. 17, 18):

Das zu druckende Gewebe wird um einen ökonomischen Druckvorgang zu erreichen, in einzelne Gewebemodule unterteilt, die sich wiederum aus Einzelmodulen zusammensetzen. Das aus den Einzelmodulen bestehende Gewebemodul sowie die Einzelmodule bilden einen Querschnitt aus einer definierten Ebene des gesamten Gewebes oder Organs mit seinen unterschiedlichen Zelltypen und Gefäßen.

Dieses Einzelmodul wird über einen Algorithmus in viele aufeinanderfolgende Ebenen (Slices) weiter unterteilt. Jede Ebene bildet dann ein zweidimensionales Druckbild mit den unterschiedlichen Zelltypen und Mustern. Durch das Drucken dieser Ebenen (Slices) entsteht wieder ein dreidimensionales Gewebe.

Entsprechend der Vorgaben des Lebermoduls wird die Druckplatte mit den Versorgungsöffnungen aus einem Polymer gedruckt. Die Versorgungsöffnungen sind die zu - und abführenden Gefäße der einzelnen Leberläppchen (Arterie, Pfortader, Vene Gallengang).

Ebenso wird die Druckfolie aus einem bioabbaubaren Polymer (bevorzugt Polyhydroxybutyrate, PHB) mit den Versorgungsöffnungen geduckt und zusammen mit der Druckplatte auf dem Druckertisch befestigt und in den Druckraum eingesetzt.

Die Behälter für Kapillartinten, Gewebetinten und Strukturtinten werden befüllt. Die Tanks für die Hepatozyten, Endothelzellen, Cholangiozyten und Epithelzellen werden angeschlossen.

Die für den jeweiligen Druck benötigten Zelltypen werden im Vorfeld von einem externen, dafür ausgestatteten und zertifizierten Labor vermehrt und für den Druckvorgang vorkonzentriert und in den jeweiligen Tanks zum Drucker transportiert.

Die Behälter für den Medium Zu - und Ablauf werden befüllt und die zu- und abführenden Schläuche an die entsprechenden Anschlüsse am Druckertischfuß angeschlossen. Versorgt wird der Zu- und Ablauf über ein Pumpensystem, welches einen Vor- und Rücklauf besitzt.

Der Druckerraum wird ebenfalls mit Medium gefüllt, welches über einen Zu- und Ablaufsystem konstant gehalten werden kann.

Der Druckvorgang kann nun gestartet werden, die Druckplatte fährt in ihre Startposition. Die Organisation aller Vorgänge wird durch dafür programmierte Algorithmen vorgenommen, die ihrerseits auch wiederum Kontollsignale vom Drucker erhalten und weiterverarbeiten.

Der Drucker druckt nun Ebene für Ebene und bildet dabei Schicht für Schicht die einzelnen Gewebebereiche aus und sinkt dabei in das Medium des Druckerraums ab, bis das Gewebemodul fertig ist. Der Druckvorgang ist so programmiert, dass nach Erreichen einer bestimmte Druckhöhe des Gewebes ein kurzer Spülvorgang eingeleitet wird, um die überschüssigen und unvernetzten Zellen auszuspülen. Dabei ist der Druck so programmiert, dass der Spülvorgang sich mit steigender Anzahl der erfolgten Druckebenen verlängert, um auszuspülende Zellen bis in den Druckraum zubefördern.

Dieser Spülprozess kann in verschiedenen Ausführungsformen stattfinden. In einer bevorzugten Ausführungsform wird einmal der aufsteigende Ast gespült, dann nach einer weiteren definierten Anzahl an Druckvorgängen der absteigende Ast. Immer alternierend, bis das Gewebemodul fertig gedruckt ist. Durch den alternierenden Spülvorgang, können die zu - und ablaufenden Gefäße gespült werden, ohne die Gefäße zu überlasten.

Nach dem das Lebermomodul fertig gedruckt wurde, kann es mit Hilfe der Druckfolie problemlos vom Drucktisch gelöst werden.

Wenn alle für das Gewebe benötigten Lebermodule sowie das Gewebe-Abschussmodul und das Gewebe-Anschlussmodul gedruckt sind, werden sie mit einem Kleber (z.B. Fibrinkleber) zu einem vollständigen Gewebe oder Organ zusammen gesetzt.

Über den im Gewebe gedruckten arteriellen und venösen Kreislauf, kann das Gewebe weiter mit Medium versorgt werden und in einem Inkubator noch mehrere Tage reifen, bevor es transplantiert wird (**Abb. 19**).

### Beispiele:

Die Beispiele 1) bis 4) entsprechen den Beispielen, die zur Erklärung unter dem Kapitel "Gegenstand der Erfindung" bereits beschrieben wurden. Sie werden anschließend durch weitere Ausführungsbeispiele ergänzt:

Die in den Beispielen aufgeführten Bezugszeichen beziehen sich insbesondere auf die konkreten Figuren.

### 1) Allgemeiner Ablauf eines Drucks - theoretische Grundlagen und bevorzugte Ausführungen

### Abbildung 15:

Die hier offenbarte Erfindung beschreibt ein Verfahren zum 3D-Druck von Organen und Geweben. Größere Druckbereiche werden in Einzelmodule aufgegliedert, unabhängig voneinander gedruckt (auf dem gleichen oder auf verschiedenen Druckern) und anschließend zum Zielorgan oder -gewebe zusammengesetzt und verklebt (z.B. mit Fibrinkleber). Zu- und abführende Gefäße der Einzelmodule werden dabei zu zu- oder abführenden Hauptgefäßen zusammengeschlossen. Das Einzelmodul ist ein bevorzugt kubisches Gebilde mit definierter Kantenlänge.

Ein Gewebe oder ein Organ sind zwar vom Aufbau gleich aber nicht identisch. Jedes Individuum unterscheidet sich, auch bei gleicher Spezies, geringfügig von einem anderen. Das äußert sich nicht nur in der Größe, sondern auch in der individuellen Entwicklung. Die Muskeln eines Sportlers zum Beispiel sind aufgrund des höheren Sauerstoffbedarfs wesentlich stärker kapillarisiert als bei einem Nichtsportler. Der Körper passt sich den Umweltbedingungen an und ist daher niemals identisch und erlebt einen ständigen Auf- und Abbau.

Entscheidend ist daher die Funktion des Gewebes und nicht die exakte anatomische Wiedergabe des Gewebes des Patienten. Daher wird hier ein Modulsystem offenbart, welches die Funktion des Gewebes ermöglicht aber auch soweit vereinfacht und systematisiert wurde, dass der 3D- Druck des Gewebes ökonomisch durchgeführt werden kann.

Der Grundbaustein des Gewebes oder des Organs ist das kubische Einzelmodul (151) (Abb.15a) von definierter Seitenlänge. Zur Versorgung des Gewebes, welches aus ein bis mehreren Zellarten bestehen kann und daher auch ein funktionelles Gewebe wie ein Organ bilden kann, wird an der einen Seite von einem aufsteigenden Gefäß (152), welches später die Arterie bildet, versorgt. Das an der gegenüberliegenden Seite befindliche absteigende Gefäß (153) bildet die Vene. Von den Gefäßen gehen jeweils fächerförmig kleinere Gefäße (154) ab, die sich bis zur Höhe des gegenüberliegenden Gefäßes auf der anderen Seite erstrecken und parallel zueinander verlaufen (Abb.15b). Die kleineren Gefäße (154) sind durch Brückengefäße (156) miteinander verbunden, welche später die Kapillaren bilden und den "Blutkreislauf" schließen und einen Mikrokreislauf bilden (Abb. 15c und 15d).

Der Mikrokreislauf stellt die kleinste Versorgungseinheit in dem gedruckten Gewebe dar. Hierbei wird über das aufsteigende Gefäß (Arterie) (152), das Medium in ein kleines Gefäß (154) geleitet, welches zum aufsteigenden Ast gehört. Von dem kleineren Gefäß strömt das Medium über die Brückengefäße (156) in ein darunterliegendes kleineres Gefäß (154), welches zum absteigenden Ast gehört und im absteigenden Gefäß (153) mündet.

Da beim Drucken der Gefäße mit der Kapillartinte viele Zellen pro Tropfen verdruckt werden und nur die Zellen an den Randbereichen in der Lage sind sich mit dem umliegenden Gewebe zu vernetzen, müssen die überschüssigen Zellen ausgespült werden. Dazu und zur Versorgung der gedruckten Zellen wird nach jeder Fertigstellung eines Mikrokreislaufs, dieser mit Medium für eine kurze Zeit durchspült. Bei jedem Spülvorgang werden die unterhalb des letzten Mikrokreislauf liegenden Mikrokreisläufe mit gespült und die Zellen versorgt. Der Fluss des Mediums spült die unvernetzten Zellen aus. Horizontal verlaufende Gefäße sind dafür nicht optimal, weshalb die gedruckten Gefäße alle in einem Winkel von 10 bis 90 Grad zur Druckerplatte verlaufen.

Die auf- und absteigenden Gefäße (2 und 3), steigen in einem Winkel von 90° an. Die Gefäßfächer (155) können in einem Winkel von 10 - 90° ansteigen. Gedruckt wird bis zur Mitte des Einzelmoduls aufsteigend, danach absteigend, sodass die kleineren Gefäße immer aufsteigend zur Druckerplatte verlaufen. So können bei den unfertig gedruckten Gefäßen die überschüssigen Zellen gut ausgespült werden. Wenn ein Mikrokreislauf geschlossen ist, kann das Medium über das absteigende Gefäß ablaufen (Abb. 15f).

Die Gefäßfächer werden in einem definierten Abstand gedruckt, wobei der Gefäßfächer des auf - und absteigenden Gefäßes abwechselnd untereinander verlaufen. Jeweils ein Gefäßfächer des aufsteigenden Gefäßes (152) und des absteigenden Gefäßes (153) werden durch Brückengefäße (156) miteinander verbunden und bilden den Mikrokreislauf (Abb. 15d). Dieser Vorgang wird so lange wiederholt, bis das Einzelmodul gefüllt ist. Zum Drucken eines Gewebes oder eines Organs, wird ein Gewebemodul (158) geduckt welches aus mehreren Einzelmodulen besteht. Die Einzelmodule werden auf der Druckerplatte, in ihrer Ausrichtung versetzt (Abb. 15g), bündig nebeneinander gedruckt, sodass sich ein Gewebemodul von definierter Seitenlänge und Dicke ergibt. Die Organmodule können zeitlich parallel auf verschiedenen Druckern gedruckt werden und mittels eines Klebers (z.B. Fibrinkleber) zu größeren Gewebe- oder Organeinheiten verbunden werden (Abb. 15e).

Um die Gewebemodule an den Kreislauf des Körpers anschließen zu können, muss insbesondere ein Gewebeabschlussmodul (158) (Abb. 15g) und ein Gewebeanschlussmodul (Abb.15h) gedruckt werden.

Das Gewebeabschlussmodul (158) besteht aus einem Gewebemodul, bei dem sich in den Einzelmodulen die auf- und absteigenden Gefäße nach oben verjüngen und so verschließen und mit mehreren Zelllagen (1510) abgeschlossen werden.

Das Anschlussmodul muss bevorzugt die einzelnen Gefäße der der Einzelmodule zu größeren Gefäßen zusammenführen und dabei die anatomischen und chirurgischen Vorgaben berücksichtigen. Dabei verlaufen die zu - und abführenden Gefäße auf unterschiedlichen Ebenen zu den Anschlussgefäßen hin. Die aufsteigenden Gefäße und die absteigenden Gefäße sind wieder durch Brückengefäße zur Versorgung des Gewebes miteinander verbunden.

### 2) Allgemeine Beschreibung des Druckvorgangs:

Das zu druckende Gewebe wird um einen ökonomischen Druckvorgang zu erreichen in einzelne Gewebemodule unterteilt, die sich wiederum aus Einzelmodulen zusammensetzen. Das aus den Einzelmodulen bestehende Gewebemodul, sowie die Einzelmodule bilden einen Querschnitt aus einer definierten Ebene des gesamten Gewebes oder Organs mit seinen unterschiedlichen Zelltypen und Gefäßen. Diese Unterteilung erfolgt mit Hilfe eines spezifischen Algorithmus. Durch das Drucken der einzelnen Ebenen (Gewebeschnitte), entsteht wieder ein dreidimensionales Gewebe.

Entsprechend der Vorgaben des Gewebemoduls, wird die Druckplatte mit den Versorgungsöffnungen aus einem Polymer gedruckt.

Ebenso wird die Druckfolie aus einem bioabbaubaren Polymer geduckt und zusammen mit der Druckplatte auf dem Druckertisch befestigt und in den Druckraum eingesetzt.

Die Tanks für Kapillartinten, Gewebetinten und Strukturtinten werden befüllt und angeschlossen.

Die benötigten Zelltypen werden in einem externen, dafür ausgestatteten und zertifizierten Labor vermehrt und für den Druckvorgang vorkonzentriert und in den Tanks zum Drucker transportiert.

Die Behälter für den Medium Zu - und Ablauf werden befüllt und die zu- und abführenden Schläuche an die entsprechenden Anschlüsse am Druckertischfuß angeschlossen. Versorgt wird der Zu- und Ablauf über ein Pumpensystem, welches einen Vor- und Rücklauf besitzt. Der Druckerraum wird ebenfalls mit Medium gefüllt, welches über einen Zu- und Ablaufsystem konstant gehalten werden kann. Der Druckvorgang kann nun gestartet werden, die Druckplatte fährt in ihre Startposition.

Zuerst werden mit der Strukturtinte die Gefäßanschlüsse auf die Druckfolie auf der Druckplatte gedruckt. Danach druckt der Drucker Ebene für Ebene und bildet dabei Schicht für Schicht die einzelnen Gewebebereiche und Gefäße aus und sinkt dabei in das Medium des Druckerraums ab, bis das Gewebemodul fertig ist. Der Druckvorgang ist dabei so programmiert, dass in immer wiederkehrenden Zyklen nach dem Erreichen einer bestimmten Höhe des Gewebes ein kurzer Spülvorgang eingeleitet wird, um die überschüssigen und unvernetzten Zellen auszuspülen. Dieser Spülprozess kann in verschiedenen Ausführungsformen vorliegen. Bevorzugt wird nach einer definierten Anzahl an Druckvorgängen einmal der aufsteigende Ast gespült, dann nach einer weiteren definierten Anzahl an Druckvorgängen der absteigende Ast. Immer alternierend, bis das Gewebemodul fertig gedruckt ist. Durch den alternierenden Spülvorgang können die zu - und ablaufenden Gefäße gespült werden, ohne die Gefäße zu überlasten.

Wenn das Einzelmodul fertig gedruckt wurde, kann es mit Hilfe der Druckfolie problemlos vom Drucktisch gelöst werden. Die gedruckten Einzelmodule werden mit einem Kleber (z.B. Fibrinkleber) zu einem vollständigen Gewebe oder Organ zusammengesetzt. Die zu- und abführenden Gefäße der Einzelmodule werden zu zu- und abführenden größeren Gefäßen vereinigt.

Über den im Gewebe gedruckten arteriellen und venösen Kreislauf kann das Gewebe weiter mit Medium versorgt werden und in einem Inkubator noch mehrere Tage reifen, bevor es transplantiert wird.

### 3) Einzelmodule am Beispiel der Leber (Abbildung 17)

Die Leber besteht aus ca. 1 - 1,5 Millionen Leberläppchen mit einem Durchmesser von 1-2 mm. In die Leber enden zwei Gefäße, die Leberarterie zur Versorgung des Gewebes mit Sauerstoff und Nährstoffen und die Pfortader, die das Blut aus Magen und Darm mit den aufgenommenen Nährstoffen und Giftstoffen transportiert. Aus der Leber heraus gehen die Venen die sich zur Hohlvene vereinen und der Gallengang.

Das Einzelmodul wird hier aus den einzelnen hexagonalen Leberläppchen (171) (Abb.17a) mit einem Durchmesser von 2 mm gebildet. Die Leberläppchen sind nebeneinander in 5 Reihen angeordnet und jeweils 5 Reihen hoch (Abb. 17b). Durch die Anordnung der Leberläppchen in den Reihen, das jede zweite Reihe um ein halbes Leberläppchen versetzt ist, lassen sie sich bei der Bildung von Organmodulen verzahnen.

Die einzelnen hexagonalen Leberläppchen haben in jeder Ecke 3 Gefäße (Arterie (172), Pfortader (174), Gallengang) und in der Mitte die Vene (175). Alle 4 Gefäße bilden wieder einen Mikrokreislauf (Abb.17b).

Das Leberläppchen ist in jeweils zu den einzelnen Eckgefäßen in 6 Segmente (176) unterteilt, die von den Mikrokreisläufen durchströmt werden und einen Gefäßfächer (Abb.17d) bilden. Die beiden zuführenden Gefäße, die Arterie und die Pfortader laufen im Mikrokreislauf parallel zueinander zur Vene hin. Ihnen entgegen kommt aus der Mitte die Vene, sie verläuft unterhalb der zuführenden Gefäße, so bilden die drei Gefäße ein Dreieck. Die Arterie und die Pfortader sind wieder mit Gefäßbrücken mit der Vene verbunden und bilden einen Mikrokreislauf. Der Gallengang befindet sich in der Mitte des Gefäßdreiecks.

### 4) Beispielhafter Druckvorgang eines Lebermoduls (Abbildung 17, 18):

Das zu druckende Gewebe wird um einen ökonomischen Druckvorgang zu erreichen, in einzelne Gewebemodule unterteilt, die sich wiederum aus Einzelmodulen zusammensetzen. Das aus den Einzelmodulen bestehende Gewebemodul sowie die Einzelmodule bilden einen Querschnitt aus einer definierten Ebene des gesamten Gewebes oder Organs mit seinen unterschiedlichen Zelltypen und Gefäßen.

Dieses Einzelmodul wird über einen Algorithmus in viele aufeinanderfolgende Ebenen (Slices) weiter unterteilt. Jede Ebene bildet dann ein zweidimensionales Druckbild mit den unterschiedlichen Zelltypen und Mustern. Durch das Drucken dieser Ebenen (Slices) entsteht wieder ein dreidimensionales Gewebe.

Entsprechend der Vorgaben des Lebermoduls wird die Druckplatte mit den Versorgungsöffnungen aus einem Polymer gedruckt. Die Versorgungsöffnungen sind die zu - und abführenden Gefäße der einzelnen Leberläppchen (Arterie, Pfortader, Vene Gallengang).

Ebenso wird die Druckfolie aus einem bioabbaubaren Polymer (bevorzugt Polyhydroxybutyrate, PHB) mit den Versorgungsöffnungen geduckt und zusammen mit der Druckplatte auf dem Druckertisch befestigt und in den Druckraum eingesetzt.

Die Behälter für Kapillartinten, Gewebetinten und Strukturtinten werden befüllt. Die Tanks für die Hepatozyten, Endothelzellen, Cholangiozyten und Epithelzellen werden angeschlossen.

Die für den jeweiligen Druck benötigten Zelltypen werden im Vorfeld von einem externen, dafür ausgestatteten und zertifizierten Labor vermehrt und für den Druckvorgang vorkonzentriert und in den jeweiligen Tanks zum Drucker transportiert.

Die Behälter für den Medium Zu - und Ablauf werden befüllt und die zu- und abführenden Schläuche an die entsprechenden Anschlüsse am Druckertischfuß angeschlossen. Versorgt wird der Zu- und Ablauf über ein Pumpensystem, welches einen Vor- und Rücklauf besitzt.

Der Druckerraum wird ebenfalls mit Medium gefüllt, welches über einen Zu- und Ablaufsystem konstant gehalten werden kann.

Der Druckvorgang kann nun gestartet werden, die Druckplatte fährt in ihre Startposition. Die Organisation aller Vorgänge wird durch dafür programmierte Algorithmen vorgenommen, die ihrerseits auch wiederum Kontollsignale vom Drucker erhalten und weiterverarbeiten.

Der Drucker druckt nun Ebene für Ebene und bildet dabei Schicht für Schicht die einzelnen Gewebebereiche aus und sinkt dabei in das Medium des Druckerraums ab, bis das Gewebemodul fertig ist. Der Druckvorgang ist so programmiert, dass nach Erreichen einer bestimmte Druckhöhe des Gewebes ein kurzer Spülvorgang eingeleitet wird, um die überschüssigen und unvernetzten Zellen auszuspülen. Dabei ist der Druck so programmiert, dass der Spülvorgang sich mit steigender Anzahl der erfolgten Druckebenen verlängert, um auszuspülende Zellen bis in den Druckraum zubefördern.

Dieser Spülprozess kann in verschiedenen Ausführungsformen stattfinden. In einer bevorzugten Ausführungsform wird einmal der aufsteigende Ast gespült, dann nach einer weiteren definierten Anzahl an Druckvorgängen der absteigende Ast. Immer alternierend, bis das Gewebemodul fertig gedruckt ist. Durch den alternierenden Spülvorgang, können die zu - und ablaufenden Gefäße gespült werden, ohne die Gefäße zu überlasten.

Nach dem das Lebermomodul fertig gedruckt wurde, kann es mit Hilfe der Druckfolie problemlos vom Drucktisch gelöst werden.

Wenn alle für das Gewebe benötigten Lebermodule sowie das Gewebe-Abschussmodul und das Gewebe-Anschlussmodul gedruckt sind, werden sie mit einem Kleber (z.B. Fibrinkleber) zu einem vollständigen Gewebe oder Organ zusammengesetzt.

Über den im Gewebe gedruckten arteriellen und venösen Kreislauf, kann das Gewebe weiter mit Medium versorgt werden und in einem Inkubator noch mehrere Tage reifen, bevor es transplantiert wird.

### 5) Eine erweiterte Variante des Drucker-Aufbaus:

Der Drucker ist aufgebaut aus:
- mehreren Druckerköpfen mit einer Anzahl von Düsen für das Droplet-Verfahren und mit einer oder mehreren räumlich steuerbaren Quellen für elektromagnetische Wellen, die zu einem in Wellenlänge, Intensität und Frequenz modulierbaren Energieeintrag führen
- die modulierbaren elektromagnetischen Wellen dienen der selektiven Aktivierung von Vernetzungsreaktionen
- einer mit Medium gefüllten verschließbaren Reaktionswanne mit Anschlüssen für Mediumwechsel, Probenahmen und nach dem Druck fakultativ geänderter Zufuhrroute für Medium oder Patientenblut, die Reaktionswanne ist aus dem Drucker entnehmbar und kann als Inkubator fungieren
- einer in der Reaktionswanne befindlichen Bodenplatte mit Anschlüssen für das entstehende vaskulare System, durch das kontinuierlich oder in Intervallen Medium strömt und zur Versorgung der gedruckten Gewebe dient
- mehreren Tanks für die erforderlichen Tinten
- verschiedenen Kapillartinten, Biotinten und Strukturtinten
- strukturelle Bereiche (z.B. Stützstrukturen, extrazellulärer Matrix oder andere nicht zelluläre Bestandteile, beispielsweise Polyhydroxyalkanoate, Fibrinogene, Collagenen, Myeline, Glycosamine, Glycolipide, Peptide, Zuckermoleküle, Fettsäuren, Schlüssel-Schloss-Moleküle, organische und anorganische Moleküle für die Reizleitung usw.)
- Eigenschaftsmoleküle (z.B. Moleküle verschiedener physiologischer Eigenschaften, beispielsweise Wachstumsfaktoren, Transkriptionsfaktoren, Rezeptormoleküle, Signalmolekülen, RNA, DNA, Differenzierungsmoleküle usw.)
- homogene Zelltypen (z.B. Myozyten, Leberzellen, Neuronen, usw.)
- Gemische aus heterogenen Zelltypen (z.B. Endothelzellen, Muskelzellen) für den Druck des vaskularen Systems
- Der Druckertisch ist während des Drucks auf - und abfahrbar, fakultativ kann stattdessen der Druckerkopf auf- und abfahrbar sein oder beides zusammen

### 6) Beispiel einer Reifung des gedruckten Gewebes im Inkubator

Der Inkubator dient der Ausdifferenzierung des gedruckten Gewebes oder Organs. Nach dem Drucken ist zwar schon ein Gewebekomplex mit hoher Zelldichte entstanden, die Zellen haben aber noch nicht ihre endgültige Gewebestruktur und Differenzierung erlangt. Das Gewebe oder Organ wird direkt in eine Kammer gedruckt, die später als Inkubator dient. Fakultativ kann das Gewebe oder das Organ auch in einen weiteren Inkubator überführt werden. Der Inkubator liefert wichtige Differenzierungsparameter, die für die Ausdifferenzierung notwendig sind, beispielsweise chemische oder molekularbiologische Stoffe oder Strömungsparameter.

Der Inkubator kann fakultativ mit Nährmedium betrieben werden und zu einem späteren Zeitpunkt außerhalb des Körpers mit dem Blutkreislauf des Patienten verbunden werden oder der Inkubator wird gleich nach dem Druck mit dem Blutkreislauf des Patienten verbunden. Beim Kontakt mit Patientenblut gelangen notwendige Differenzierungs- und Wachstumsfaktoren zu den gedruckten Strukturen und die Zellen können an den Körper adaptieren.

### 7) Beispielhafter Aufbau des Druckerkopfs (Abbildung 19)

Der Druckerkopf besteht aus einem *ersten Teilbereich* für den Droplet - Druck der Biotinten, Kapillartinten und Strukturtinten. Das Droplet-Verfahren läuft in der Regel über über Piezo-Düsen oder Bubble-Düsen. Der Druckvorgang ist im Ablauf und der erzeugten Anordnung der Tropfen ähnlich dem Cyan/Magenta/Yellow/Schwarz/Grau/Hellgrau/Light Magenta/Light Cyan - System von Fotodruckern. Anstelle der Farbtinten steht die erforderliche Anzahl von Biotinten, Kapillartinten und Strukturtinten.

In einem *zweiten Teilbereich* besitzt der Druckerkopf eine oder mehrere ungerichtete oder gerichtete Quellen für elektromagnetische Wellen. Die gerichteten Quellen erzeugen in ihrer Gesamtheit eine selektive Energiequelle, die bezüglich Wellenlänge, Intensität und Frequenz modulierbar ist. In der Regel dienen als Quellen für elektromagnetische Wellen UV-Lampen, Leuchtdioden, Laser oder Screens. In der Regel verwendete elektromagnetische Wellen sind ultraviolettes Licht, sichtbares Licht und Wellenlängen im Infrarotspektrum.

Die Funktionseinheiten des Druckerkopfs für das Dropletverfahren und für das Verfahren zur Bereitstellung der elektromagnetischen Wellen können auch voneinander getrennt angeordnet sein. Ebenfalls kann der Druckerkopf aus einer Reihe von Modulen solcher Einheiten aufgebaut sein.

### 8) Weiterer alternativer Aufbau der Reaktionswanne mit Bodenplatte, Zufuhr von Medium (Abbildung 1, 2,3,4,5,6,7):

Im Druckraum des Druckers befindet sich ein auf- und abfahrbarer Druckertisch mit einer Anzahl von Anschlüssen für die Durchströmung der gedruckten vaskularen Strukturen mit Medium.

Außerhalb des Druckraums sind die Anschlüsse mit einem externen System zur Aufrechterhaltung von bestimmten Zellkulturparametern verbunden. Dazu gehören u.a. Temperatur, pH-Wert, CO₂-Gehalt, O₂-Geahlt, Nährstoffe, Wachstumsfaktoren usw.. Da auch die nicht vernetzten Zellen innerhalb der gedruckten vaskularen Strukturen in Form von Zelltrümmern oder als ganze Zellen ausgespült werden, muss das in den Druckraum abfließende Medium bevorzugt in seinen Parametern kontrolliert und gegebenenfalls gereinigt oder getauscht werden.

Während des Drucks des vaskularisierten Gewebes wird Medium durch die Anschlüsse in die darauf aufgedruckten vaskularen Strukturen geleitet. Dies dient einerseits der Ernährung des entstehenden Gewebes und andererseits dem Freispülen der Hohlstrukturen des vaskularen Systems. Das Medium tritt durch die Anschlüsse des Druckertischs in die vaskularen Strukturen, durchströmt das soweit gedruckte vaskularisierte Gewebe und sickert oberhalb an der dem Druckerkopf zugewandten Seite an den noch offenen Hohlräumen des vaskularen Systems wieder aus. Von dort fließt das Medium entweder passiv zurück in den Druckerraum oder wird während des Druckprozessen aktiv von der Druckoberfläche entfernt (z.B. abspülen bzw. absaugen).

Der Druckertisch senkt sich während des Druckvorganges immer weiter in den Druckraum, sodass das gedruckte Gewebe mit dem Medium im Druckraum umspült ist. Ca. 1 - 5 mm des obersten Bereichs des Gewebes ragt aus dem Medium hervor. Da die Thiol-en - Reaktionen relativ O₂-unempfindlich ablaufen, ist es in der Regel nicht erforderlich, die luftgefüllten Bereiche der Reaktionswanne mit Inertgas (z.B. Stickstoff, Argon) zu füllen.

### 9) Bereitstellung der notwendigen digitalen Informationen für die 3D-Drucker - Software

Die digitalen Daten über den zellulären Aufbau von Geweben und Organen werden aus medizinischen Bildgebungsverfahren erhalten und zu 3D-Druckdaten programmiert. Zu diesen Verfahren gehören beispielsweise das MRT - Verfahren oder das "DISCO-Transparenz" - Verfahren. Die Verfahren zur Ermittlung dieser Daten sind dem Fachmann bekannt. Aus diesen Daten wird mittels eines speziellen Algorithmus eine Anzahl erforderlicher Module zum ökonomischen Druck erforderlicher Zelltypen sowie der erforderlichen Größe und Abstand von Kapillaren, venösen und arteriellen Gefäßen berechnet. Für einen ökonomischen Druck ist es nicht erforderlich, die biologischen Strukturen im Original nachzudrucken. Der Aufbau von Einzelmodulen wurde bereits unter 1) bis 4) beschrieben.

### 10) Neuartige Druckersoftware:

Die - nicht beanspruchte - Druckersoftware verarbeitet die Informationen über die zu druckenden Gewebereiche und gibt diese zur Ausführung an den Druckerkopf und den Druckertisch weiter. Im Gegensatz zu herkömmlichen 3D-Drucker-Programmierungen werden hier sowohl die Droplet-Technologie (Druckmuster der spezifischen Tinten und Tropfengröße) als auch im Fall einer gerichteten Quelle für elektromagnetische Wellen eine modulierte Lasertechnologie (geforderte Wellenlängen, Energie-Intensitäten und Frequenzen, räumliche Aussteuerung) miteinander kombiniert und aufeinander abgestimmt. Eine zentrale Rolle dabei spielt die räumliche und zeitliche Koordinierung der Tropfenabgabe spezifischer Tintentypen in auf die Reaktionswanne Druckplatte mit dem Gewebe mit der zu deren Vernetzung notwendigen räumlichen und zeitlichen Bereitstellung elektromagnetischer Wellen in einer tintenspezifischen Wellenlänge, Frequenz und Intensität. Im Mittelpunkt steht die selektive Vernetzung von Endothelzellen und von Muskelzellen innerhalb eines Tintentropfens aus einem Zellgemisch heraus zu vaskularen Strukturen (Abschnitt "Verwendete Schutzgruppen und Photoinitiator")

### 11) Reifung eines gedruckten Gewebes und Organs bis zur Transplantation:

Der Inkubator mit Gewebe oder Organ besitzt Anschlüsse für die ex vivo Anknüpfung an den Blutkreislauf des Patienten oder an ein Medium lieferndes System. Durch eine Inkubation ausgehend von einigen Stunden bis einigen Tagen wird das Zellgefüge des 3D Drucks weiter ausdifferenziert. Besonders durch die Verbindung des Gefäßkreislaufs des Drucks mit dem Blutkreislauf des Patienten werden wichtige Entwicklungsfaktoren an die Zellen geliefert. Die Endothelzellen und Muskelzellen der gedruckten vaskularen Strukturen benötigen außerdem zur Differenzierung einen spezifischen Gefäßinnendruck durch das Blut oder das Medium und eine spezifische Strömungsgeschwindigkeit.

Während der Reifungsphase ist es möglich, über Anschlüsse im Inkubator spezifische physiologische Werte zu überprüfen und damit die Reifung des Organs oder Gewebes zu überwachen. Wenn die physiologischen Werte des gedruckten Organs oder Gewebes in einem bestimmten Rahmen die physiologischen Werte eines natürlichen Organs erreichen, kann die Transplantation in den Körper des Patienten erfolgen.

### 12) Beispielhafter Aufbau der neuartigen niedrigviskosen Tinten:

Der prinzipielle Aufbau der Tinten besteht aus niedrigviskosen, kurzkettigen Vernetzungsmolekülen, aus Photoinitiatoren und aus Zellen. Die kurzkettigen Vernetzungsmoleküle besitzen mindestens eine in der Regel 2 oder mehr funktionelle Gruppen, die in der Regel aus Allylgruppen oder Thiolgruppen bestehen und Polymerisations- oder Additionsreaktionen durchlaufen.

Häufig verwendete Vernetzungsmoleküle sind methacrylatmodifizierte und thiolmodifizierte Moleküle, z.B. methacrylatmodifizierte oder thiolmodifizierte Peptidketten, Polyhydroxyalkanoate, oder Hyaluronsäuren. Die Biotinten und Strukturtinten sind entweder überwiegend aus methacrylatmodifizierten Molekülen (radikalische Polymersierung oder Addition) aufgebaut oder auch aus einer Mischung von methacrylatmodifizierten Molekülen mit thiolmodifizierten Molekülen (Thiol-en Reaktionen). Die Kapillartinten hingegen sind in der Regel aus reinen thiolmodifizierten Vernetzungsmolekülen aufgebaut, die nur an den Randbereichen des Tropfens in Wechselwirkung mit den allylmodifizierten Vernetzungsmolekülen der Biotinten und Strukturtinten vernetzen.

In der Regel werden Photoinitiatoren verwendet, die entweder im Bereich von ca. 380 nm arbeiten oder mit Photoinitiatoren, die im Infrarotbereich liegen. Bei Photoinitiatoren im Infrarot-Bereich handelt es sich um Stereo-Energiequellen ähnlich der Multiphotonenstereolithographie (nichtlineare Mehr-Photonen-Absorption), bei der verschiede Energiequellen überlagert werden müssen, um eine Reaktion auszulösen.

Beim Druck von Zellen mit selektiven Faktoren (z.B. spaltbare Schutzgruppen für Thiolgruppen) werden zusätzliche Photoinitatoren unterhalb von 380 nm verwendet.

Beispiele für verwendete Photoinitiatoren sind DAROCUR 1173, IRGACURE 2959 UND 369, *(Ru(phen)₃*/*Iod*/*(TMS)₃Si-H PIS) und Photoinitiator 183 aus "*P. Xiao et al./Progress in Polymer Science 41 (2015) 32 - 66, S. 46". Photoinitiatoren für IR-Anregungen sind *H-Nu-IR 780* und *H-Nu-IR* 815. Es hat sich herausgestellt, dass eine Reihe von Photoinitiatoren aus dem Bereich des sichtbaren Lichts ebenfalls durch Multiphotonen aktivierbar sind.

### 13) Bereitstellen von Zellen, die zum Druck erforderlich sind:

Zellen für das hier offengelegte neuartige 3D-Druckverfahren können auf verschiedene Weise bereitgestellt werden. Der Ursprung der Zellen hängt dabei vom beabsichtigten Verwendungszweck des damit gedruckten Gewebes oder Organs ab.

Der einfachste Weg, Zellen zu generieren, ist die Verwendung von käuflichen Zell-Linien. Mit solchen Zellen lassen sich Druckparameter und Kultivierungsparameter optimieren, da man für diese Tests nicht die relativ kostspieligen induzierten Stammzellen (iPS) oder aus Organen und Geweben isolierte Zellen verwenden muss.

Eine weitere Quelle für Zellen ist die Isolierung bestimmter Zelltypen aus Organen und Geweben. Neben tierischen Zellen für die Forschung wird dieser Prozess auch schon vielfältig in der regenerativen Medizin eingesetzt, beispielsweise die Isolierung von Bandscheiben-Knorpelzellen aus Prolaps-Material von Bandscheibenoperationen. Das Prolaps-Material wird aufgelöst und die Zellen werden daraus isoliert, vermehrt und u.a. auch im 3D-Druck von Geweben eingesetzt.

Die wohl anspruchsvollste Quelle für Zellen ist die Generierung von induzierten Stammzellen (iPS). Hierfür existieren Vorgehensweisen verschiedener etablierter Arbeitsgruppen. iPS-Zellen sind für den Druck menschlicher Gewebe und Organe erforderlich, die anschließend in den menschlichen Körper implantiert werden sollen. Aber auch in der Zellkultur als Testmaterial für individualisierte Medikamente sind diese Zellen und die daraus gedruckten Strukturen einsetzbar. iPS-Zellen werden in der Regel aus Hautzellen des Spenders gezüchtet. Die Hautzellen werden zu iPS-Zellen defifferenziert, vermehrt und in die zum Druck erforderlichen Zelltypen differenziert. Diese Vorgänge sind für eine breite Anwendung noch wenig etabliert, stellen aber die Zukunft bei der Herstellung von menschlichen autologen Implantaten für Organe und Gewebe dar.

Weitere Quellen für Zellmaterial sind für spezifische Zwecke gezüchtete Zelllinien, die in der Regel auf verschiedene Weise modifiziert wurden (z.B. auf DNA-Basis, auf RNA- oder Proteinbasis).

Prinzipiell kann jeder Zelltyp (primär oder Zelllinie) gezüchtet und mit dem hier offenbarten Verfahren gedruckt werden.

### 13) Beispielhafte Tinten für den Druck mit Zellen, die einen selektiven Faktor enthalten (Abb. 13, 14):

Eine Möglichkeit, Kapillartinten nur im Randbereich zu vernetzen, ist der Einsatz von Zellgemischen aus Endothelzellen und Muskelzellen, die innerhalb eines Tropfens selektiv miteinander vernetzt werden. Um von einem gerichteten Laserstrahl selektiv aktivierbar zu sein, benötigen diese Zelltypen einen spezifischen selektiven Faktor. Dieser selektive Faktor ist beispielsweise eine spaltbare Schutzgruppe auf einer funktionellen Thiolgruppe, die sich auf der Zellmembran des betreffenden Zelltyps befindet. Durch die Verwendung verschiedener Schutzgruppen, die sich bei unterschiedlichen Wellenlängen spalten lassen, können die verschiedenen Zelltypen in ihrer Vernetzungsreaktion voneinander getrennt werden. In der Regel besteht die Kapillartinte bei dieser Anwendung aus thiolmodifizierten Vernetzungsmolekülen mit Schutzgruppe, die an der Zellmembran verankert sind und aus freien thiolmodifizierten, geschützten Molekülen in der Tinte. Zusätzlich befinden sich allylmodifizierten Moleküle in der Tinte, um eine Thiol-en - Vernetzungsreaktion zu ermöglichen.

### 14) Beispielhafter Aufbau der Tinten und selektiver Faktor (Abb. (Abb. 13, 14) :

Die lichtabhängige Thiol-en - Reaktion erfolgt innerhalb des vom Laser festgelegten räumlichen Vernetzungsbereichs selektiv über selektive Faktoren. Diese selektiven Faktoren bestehen beispielsweise aus lichtspaltbaren Schutzgruppen, die die Thiolgruppen blockieren. Aufgrund ihres unterschiedlichen molekularen Aufbaus sind diese Schutzgruppen durch verschiedene Wellenlängen des Lichts spaltbar. Es werden solche Schutzgruppen auswählt, die sich im Bereich ihres Anregungsspektrums nicht überschneiden.

Um in einem vom Laser getroffenen Bereich nur bestimmte Zelltypen aus einem Gemisch heraus selektiv zu vernetzen, andere aber nicht, müssen an den betreffenden Zellen die Schutzgruppen an den Thiolgruppen gespalten werden. Somit sind nur diese Zellen und diese Vernetzungsmoleküle einer Thiol-en - Reaktion zugänglich. Anschließend wird in diesem Beispiel die Thiol-en - Vernetzungsreaktion durch einen Photoinitiator gestartet, dessen Anregungsspektrum oberhalb der Wellenlänge aller verwendeten Schutzgruppen liegt, um diese nicht versehentlich zu spalten (Beispiele siehe unten).

### 15) Druck des vaskularen Systems mit Zellen die einen selektiven Faktor enthalten:

Da der Durchmesser der feinen Kapillaren zwischen den Arterien und Venen nur wenige Mikrometer beträgt, die Tropfen im Droplet-Verfahren aber größer sind, wird der innere einlagige Ring aus Endothelzellen aus einem Tropfen einer Zellmischung heraus selektiv vernetzt. Ebenso wird der diesen Ring umgebende Ring aus Muskelzellen aus dem Zellgemisch heraus selektiv vernetzt. Hierfür tragen die Endothel- bzw. die Muskelzellen als selektiven Faktor jeweils zu unterschiedlichen Wellenlängen spaltbare Schutzgruppen (siehe Beispiele unten).

Nach einer selektiven Spaltung der Schutzgruppen am entsprechenden Zelltyp kann bei den entschützten Zellen die Thiol-en - Reaktion ablaufen. So werden zunächst durch einen ersten Laserstrahlim Tropfen ca. 5 - 7 Endothelzellen und die dazwischen liegenden geschützen thiolmodifizierten Vernetzungsmoleküle zu einer ringförmigen Struktur entschützt und anschließend durch einen zweiten Laserstrahl untereinander ringförmig vernetzt. Der zweite Laserstrahl dient der Anregung eines Photoinitiators und liegt oberhalb der Wellenlänge von den ersten und dritten Laserstrahl.

Dadurch entsteht ein einlagiger Endothelring mit einem Innendurchmesser von ca. 10 Mikrometern, dessen innere Fläche aus nicht vernetzten, geblockten Zellen besteht. Anschließend werden durch einen dritten Laserstrahl die dem Endothelring benachbarten äußeren Muskelzellen und geschützten thiolmodifizierten Vernetzungsmoleküle entschützt und mit einem zweiten Laserstrahl untereinander zu einem Außenring vernetzt. Während die ersten und dritten Laserstrahlen bei unterschiedlichen Wellenlängen die entsprechenden Schutzgruppen spalten, führt der zweite Laserstrahl zur Aktivierung eines Photoinitiators, der die Thiol-en - Reaktionen startet.

Die unvernetzten Zellen im inneren der Kapillaren werden durch einen Laserstrahl höherer Energie (vierter Laserstrahl) zerstört und die entstehenden Zelltrümmer vom einströmenden Medium ausgespült.

### 16) Verwendete Schutzgruppen und Photoinitiatoren bei Tinten mit selektiven Faktoren (Abb. 13, 14):

Während für die Vernetzung von Tinten mit homogenen Zelltypen der Reaktionstyp frei gewählt werden kann und unselektiv abläuft, besitzen die verschiedenen Zelltypen mit heterogenen Tinten Thiol-en - Reaktionspartner mit einer spezifischen Schutzgruppe, die zur entsprechenden Wellenlänge spaltbar ist. Dabei dürfen sich die Kurven der notwendigen Aktivierungsenergien nicht überlappen (Abb. 13).

Für die Vernetzung der Endothelzellen bzw. der diese umgebenden Muskelzellen wurden folgende UV-spaltbare Schutzgruppen verwendet:
*325 nm: (7,8-Bis(carboxymethoxy)-cumarin-4yl)methoxycarbonyl für Muskelzellen*
*400 nm: α Carboxy-4methoxy-2-nitrobenzyl für Endothelzellen*
(Quelle: Wellenlängenselektiv abspaltbare photolabile Schutzgruppen für Thiole, Nico Kotzur, 2009)

Zunächst wird die Schutzgruppe mit der höheren erforderlichen Wellenlänge abgespalten, um die andere Schutzgruppe nicht versehentlich zu aktivieren. Für die eigentliche Thiol-en - Reaktion wird ein Photoinitiator *(Ru(phen)₃*/*Iod*/*(TMS)₃Si-H PIS)* verwendet, dessen Anregungsspektrum bei *532 nm* liegt, um die darunter liegenden Schutzgruppen nicht versehentlich zu spalten. Ebenfalls einsetzbar ist der *Photoinitiator 183 (*P. Xiao et al./Progress in Polymer Science 41 (2015) 32 - 66, S. 46), der bei *473 nm* angeregt wird.

In einem ersten Schritt wird die Schutzgruppe an den Thiolgruppen der Endothelzellen bei 400 nm abgespalten (erster Laserstrahl). In diesem Bereich sind die Thiolgruppen der Muskelzellen noch geschützt. Danach wird der Photoinitiator bei 532 nm aktiviert und eine Thiol-en - Reaktion an den entschützten Gruppen ausgelöst (zweiter Laserstrahl). Durch die räumliche Führung der beiden nacheinander folgenden Laserstrahlen (erst 400 nm, dann 532 nm) werden in der Regel 5 bis 12 Endothelzellen ringförmig miteinander vernetzt.

In einem zweiten Schritt werden bei 325 nm die Schutzgruppen an den Thiolgruppen der Muskelzellen abgespalten Laserstrahl 3). Da die räumliche Führung der Laserstrahlen für den Bereich der Endothelzellen (400 nm und 532 nm) den Bereich des die Endothelzellen umgebenden Muskelringes nicht trifft, liegen dort die Endothelzellen der Zellmischung noch mit Schutzgruppen vor und können an der Thiol-en - Reaktion der entschützten Thiolgruppen der Muskelzellen nicht teilnehmen. Anschließend erfolgt durch die Aktivierung des Photoinitiators bei 532 nm eine Vernetzung der im Ring befindlichen Muskelzellen zweiter (Laserstrahl).

Auf diese Weise kann sichergestellt werden, dass überwiegend nur der gewünschte Zelltyp in die entsprechende Struktur eingebaut wird.

Für weitere benötigte selektive Faktoren stehen zusätzliche Schutzgruppen für Thiolgruppen zur Verfügung (Abb.13), z.B. die bei 436 nm spaltbare Gruppe ((7Bis(carboxymethyl)amino)-cumarin-4-yl)methoxycarbonyl ). Der Vernetzungsablauf erfolgt analog wie oben beschrieben. (Quelle: Wellenlängenselektiv abspaltbare photolabile Schutzgruppen für Thiole, Nico Kotzur, 2009)

Die nicht vernetzen Zellen im Inneren der Kapillaren werden durch einen vierten Laserstrahlhöherer Energie zerstört und die Trümmer vom Medium ausgespült.

### 17) Beispielhafte Herstellung von autologen Patientenzellen mit einem selektiven Faktor

Die vom Patienten entnommenen Zellen (z.B. Hautzellen) werden zu induzierten Stammzellen (iPS) umgewandelt, vermehrt, in einzelne Fraktionen gesplittet und danach in die erforderlichen Zelltypen differenziert. Diese Vorbereitung erfolgt unabhängig vom Druckprozess und wird hier nur beispielhaft erwähnt, um den Weg zu echten 3D-Organen zu beschreiben. Dieser Vorgang kann Wochen bis Monate dauern, je nachdem Komplexität des zu druckenden Organs. Nach Produktion der erforderlichen Zelllinien können diese eingefroren und gelagert werden, bis der Druck erfolgen soll. Man kann eingefrorene Zellen auftauen, mit dem selektiven Faktor hybridisieren und drucken. Schonender für die Zellen ist eine Inkubationszeit nach dem Auftauen in der Zellkultur für 24 Stunden und eine anschließende Hybridisierung mit ihrem spezifischen selektiven Faktor.

In der Regel ist der selektive Faktor mit einem Ankermolekül für die Verankerung in die Zellmembran verbunden. Die verwendeten Ankermoleküle sind in der Regel universell für alle Zelltypen einsetzbar. Überraschenderweise hat sich gezeigt, dass perfluorierte Ankermoleküle am wirksamsten sind. Erfahrungsgemäß binden diese Moleküle innerhalb von 2 Minuten an 100% der beteiligten Zellen. Als Ankermoleküle können aber auch lipophile Moleküle, Peptide, Ionen oder Antikörper fungieren.

### 18) Vorbereitung der Zellen mit selektivem Faktor zum Druck:

Kurz vor dem Druck werden die Zellen des Kapillarsystems mit ihrem spezifischen selektiven Faktor hybridisiert: Die verwendeten Endothelzellen und Muskelzellen werden zunächst getrennt in der Zellkultur auf den Druck vorbereitet und mit ihrem jeweiligen selektiven Faktor hybridisiert. Anschließend werden sie in den Kapillartinten zusammengeführt.

### Weitere Ausführungen zu den Abbildungen:

### Abbildung 1:

### Abb. 1: Beispielhafter schematischer Druckeraufbau und Funktionsweise:

Grundlage der Erfindung ist ein herkömmlicher fotorealistischer Piezodrucker.

Neuartig offenbart sind Modifikationen der Tintenzuführung (1-5) und der Aufbau des Druckertisches (12, 13, 14, 15).

Der Drucker ist aufgebaut aus den Tintenversorgungseinheiten (1 - 5) dem Piezodruckerkopf (15), dem Druckertisch (13) mit Medium gefülltem Druckraum (14) und dem Motor des Drucketisches (12). Zusätzlich befindet sich ein Mediumtank (8) zur Versorgung des Gewebes mit zuführenden Schläuchen (9) und ein Abfalltank (11) und mediumabführenden Schläuchen (10) am Drucker.

Die einzelnen Komponenten sind von einem Gehäuse (6) umgeben. Außerhalb des Gehäuses befindet sich die zentrale Steuereinheit/Computer/Software (7), die mit den Komponenten 1 - 5, 15 und 12 kommuniziert (gestrichelte Linien).

### Abbildung 2 - 7:

### Abb. 2 - 7: Beispielhafter Aufbau Druckertisch und Funktionsweise:

Im Folgenden wird ein neues System eines Druckertischs offenbart. Der Drucktisch besteht im Wesentlichen aus 5 Teilen:

Einer Druckplatte (27), einem Druckertisch (13) mit Versorgungsanschlüssen, der mittels eines Schrittmotors (23) und einer Mikrometerschraube (22) im Druckraum abgesenkt werden kann.

Die Druckplatte (27) wird mittels eines 3D Druckers (FDM, SLS, SLM SLA, oder andere Drucker) individuell für das Organ oder Organoid gedruckt, da die Öffnungen zur Versorgung des Gewebes unterschiedlich sein können. Der Unterschied kann in der Positionierung, Anzahl und Durchmesser begründet sein.

Die Druckplatte (27) aus einem biokompatiblen Polymer verfügt über die Anschlussöffnungen für den Medium Zu- und Ablauf (21), von denen sich individuell für das Gewebe ein Leitungssystem innerhalb der Platte erstreckt und in mehreren Öffnungen für die Versorgung des Gewebes an der Oberfläche münden. Die Öffnungen können unterschiedlich positioniert sein und auch unterschiedliche Durchmesser besitzen. Auf diese Öffnungen werden dann die Gefäße zur Versorgung des Gewebes gedruckt.

Die Druckerplatte wird auf den Drucktisch (13) geschraubt, der Drucktisch besteht aus einem magnetischem Material und verfügt über Bohrungen in denen die Anschlussstücke (21) für den Zu - und Ablauf des Mediums gesteckt werden. Unterhalb der Druckerplatte befindet sich der Drucktischfuß (25), in dem der Stift der Mikrometerschraube reibungsarm eingepasst ist, sowie die Anschlüsse (z.B. Lueranschlüsse) für den Zu - und Ablauf (21) des Mediums. Die Anschlussstücke des Zu- und Ablaufs an der Oberseite des Drucktischfußes (25), sind in einer bevorzugten Ausführungsform, als Zapfen konzipiert und passen exakt in die Bohrungen des Drucktischs. An der Oberseite des Drucktischfußes ist eine Magnetplatte angebracht. Über Kraft des Magneten, wird der Drucktisch und der Drucktischfuß miteinander verbunden und somit wird eine Verbindung von den Anschlüssen für den Zu -und Ablauf zur Druckplatte hergestellt. Dichtungsringe in den Bohrungen verhindern das Auslaufen von Medium. Über die Magnetverbindung kann auch das gedruckte Gewebe samt Druckplatte und Drucktisch problemlos von den anderen festinstallierten Komponenten getrennt und auf eine andere Versorgungseinheit gesteckt werden.

Über den Stift der Mikrometerschraube (22) und des Schrittmotors (23), kann der Drucktisch samt Druckerplatte im Druckraum (Raum oberhalb der Silikonmatte) abgesenkt werden. Der Druckraum besteht aus einer Bodenplatte (28) und einer Wandung (29) aus z.B. Edelstahl, und bildet somit einen Raum, der mit Medium über Zuführungen befüllt werden kann. In dieses Medium wird das gedruckte Gewebe abgesenkt um es zu ernähren und vor Austrocknung zu schützen. Durch Absenkung der gedruckten Bereiche, bleibt die Druckebene auf konstanter Höhe. Durch einen Überlauf, wird ein Überlaufen durch Verdrängung des Mediums verhindert.

In einer besonderen Ausführungsform, befindet sich oberhalb des Druckertischfußes (25) eine Silikonmatte (210) zur Dichtung.

Diese wird über den Druckertischfuß (25) gespannt, wobei die Zapfen für den Zu - und Ablauf freibleiben und über den Rand der Bodenplatte des Druckraums reichen. Durch das Aufschrauben der Druckraumwand (29), auf die Bodenplatte für den Druckraum (28) wird zum einen der Druckraum abgedichtet und die Unterseite des Drucktischfußes mit dem Stift der Mikrometerschraube vom Druckraum getrennt. Durch die hohe Flexibilität der Silikonmatte, ist die Auf - und Abbewegung des Drucktischs weiterhin problemlos möglich.

In einer weiteren Ausführungsform wird zum Abdichten des Druckraums anstelle der Silikonmatte, eine Dichtung zwischen dem Stift der Mikrometerschraube (22) und der Bodenplatte für den Druckraum (28) angebracht (z.B. Gleitringdichtung).

In weiteren Ausführungsformen können andere Konstrukte verwendet werden, um den Druckraum abzudichten.

### Abbildung 8:

### Abb. 8: Beispielhafter Aufbau Druckerkopfversorgungseinheit und Funktionsweise:

In der hier neuartig offenbarten Druckkopfversorgungseinheit werden die angezüchteten Zellen aus dem Zelltank (82) über eine Pumpe (84) in den Zellkonzentrator (86) gepumpt, in dem die Zellkonzentration erhöht und überschüssiges Medium abgeführt (89) wird. Über einen Zellzähler (88) wird eine definierte Zellkonzentration weiter geleitet zu einer Mischeinheit (87), in die auch Tintenkonzentrat eingeleitet wird. Das Tintenkonzentrat wird aus dem Vorratsbehälter (1) über eine Pumpe mit Messeinheit (85) in einem definierten Volumen in die Mischeinheit (87) befördert. In dieser Mischeinheit (87) wird die Tinte für die Druckerköpfe nach Vorgaben aus dem Druckprogramm angemischt und über einen Kontroll-Zellzähler in den zum Piezodruckerkopf (811) führenden Schlauch geleitet.

Die Anzahl der Druckerkopfversorgungseinheiten richtet sich nach der Anzahl der benötigten Biotinten und kann variieren.

Die Druckerkopfversorgungseinheit wird einerseits vom Algorithmus des Druckers gesteuert, um die erforderliche Tintenzusammensetzung zu mischen, andererseits besitzt sie eine Reihe von Sensoren, die dem Druckeralgorithmus Informationen über die Eigenschaften der gegenwärtig gemischten Tinten liefern, auf die der Algorithmus reagiert.

### Abbildung 9 u. 10

### Abb. 9 u. 10: Bevorzugte Bauausführungen des Zellkonzentrators (86) zur Aufkonzentrierung der Zellen durch Dekanter

Zur Aufkonzentrierung der Zellen kommen als Zellkonzentrator (86) bevorzugt folgende Verfahren zum Einsatz, die sich durch einen permanenten Betrieb auszeichnen. Die Aufkonzentrierung der Zellen wird durch die Reduzierung der flüssigen Phase, des Mediums, erreicht. Die Trennung von fester und flüssiger Phase kann zum einen durch die Zentrifugalkraft erfolgen oder durch Filtereigenschaften.

In diesen Bauausführungen wird zur Aufkonzentrierung der Zellen, eine Durchflusszentifuge oder ein Dekanter verwendet. Beide Systeme arbeiten nach dem gleichen Prinzip mit Hilfe der Zentrifugalkraft.

In einer Zentrifuge erfolgt die Trennung der Feststoff- und Flüssig-Phase mit Hilfe der Zentrifugalbeschleunigung. In der sich drehenden Trommel der Zentrifuge bewegen sich die Feststoffteilchen, die eine höhere Dichte aufweisen und somit schwerer als die Flüssigkeit sind, mittels Zentrifugalkraft nach außen. Sie bilden ein Sediment an der inneren Wand der Zentrifugentrommel.

Die Dekantertrommel hat eine zylindrisch-konische Form und rotiert mit einer Drehzahl, die auf die jeweilige Trennaufgabe abgestimmt ist. In der Trommel erreicht das Produkt die volle Umfangsgeschwindigkeit und legt sich als zylindrischer Ring an den Trommelmantel der Zentrifuge an. Die im Produkt enthaltenen Feststoffe setzen sich aufgrund der höheren Dichte unter dem Einfluss der Zentrifugalkraft an der Trommelinnenwand ab. Die Länge des zylindrischen und der Kegelwinkel des konischen Trommelteils lassen sich bei der Herstellung des Dekanters an die jeweilige Trennaufgabe anpassen.

### Abbildung 11 u. 12

### Abb. 11 u. 12: Bevorzugte Bauausführungen des Zellkonzentrators (86) zur Aufkonzentrierung der Zellen durch dynamische Filtration

Bei der Cross-Flow-Mikrofiltration wird die Filtermembran während des Filtrationsvorganges von dem Medium mit den Zellen überströmt, sodass zwei Hauptstromrichtungen orthogonal zueinander bestehen. Die Hauptstromrichtungen sind der Filterstrom durch das Filtermembran und die Überströmung parallel zur Filtermembran. Dem Aufbau des Filterkuchens während des Filtrationsvorganges wird durch die Überströmung der Suspension entgegengewirkt, sodass das Zellkonzentrat abfließen kann. Die Effektivität der Filtereigenschaft ist durch die Anzahl der Poren und Porengröße in der Filtermembran variable. Besonders dafür geeignet sind Hohlfasern (Kapillarmembran oder auch Hohlfäden genannt), deren Leistungsfähigkeit noch durch den Pinch-Effekt verstärkt wird. Eine übliche Hohlfaser hat einen Innendurchmesser von ca. 1,5 mm (3,0 mm bis 0,1 µm möglich) und eine Porengröße von 200 bis 5 nm (2000 nm bis 1,0 nm möglich). Je nach Anwendung werden hunderte bis tausende Kapillaren in Modulen zusammengefasst und vergossen (Hohlfasermodule). Mit Hilfe einer Zirkulationspumpe wird das unfiltrierte Produkt solange durch die Kapillaren zirkuliert, bis die Trubstoffe im Retentat so konzentriert sind, dass eine Entleerung und Reinigung erforderlich wird. (Ripperger 1992), (Melin & Rautenbach 2007).

### Abbildung 13

selektive Faktoren - spaltbare Schutzgruppen für Thiolgruppen zu den Wellenlängen von 325nm, 400 nm und 436 nm (Quelle: Wellenlängenselektiv abspaltbare photolabile Schutzgruppen für Thiole, Nico Kotzur, 2009)

### Abbildung 14

Zellen mit selektivem Faktor. Helle Zellen zeigen Endothelzellen, dunkle Zellen zeigen Muskelzellen. Beide Zelltypen liegen als Gemisch in einem Kapillartintentropfen vor und können selektiv durch 2 Laserstrahlen mit verschiedenen Wellenlängen zur Vernetzungsreaktion aktiviert werden.

### Abbildung 15

### Abb. 15: Aufbau eines Gewebemoduls:

Der Grundbaustein des Gewebes oder des Organs ist das kubische Einzelmodul (151) (Abb.15a) von definierter Seitenlänge. Zur Versorgung des Gewebes, welches aus ein bis mehreren Zellarten bestehen kann und daher auch ein funktionelles Gewebe wie ein Organ bilden kann, wird an der einen Seite von einem aufsteigenden Gefäß (152), welches später die Arterie bildet, versorgt. Das an der gegenüberliegenden Seite befindliche absteigende Gefäß (153) bildet die Vene. Von den Gefäßen gehen jeweils fächerförmig kleinere Gefäße (154) ab, die sich bis zur Höhe des gegenüberliegenden Gefäßes auf der anderen Seite erstrecken und parallel zueinander verlaufen (Abb. 15b). Die kleineren Gefäße (154) sind durch Brückengefäße (156) miteinander verbunden, welche später die Kapillaren bilden und den "Blutkreislauf" schließen und einen Mikrokreislauf bilden (Abb. 15c und 15d).

Der Mikrokreislauf stellt die kleinste Versorgungseinheit in dem gedruckten Gewebe dar. Hierbei wird über das aufsteigende Gefäß (Arterie) (152), das Medium in ein kleines Gefäß (154) geleitet, welches zum aufsteigenden Ast gehört. Von dem kleineren Gefäß strömt das Medium über die Brückengefäße (156) in ein darunterliegendes kleineres Gefäß (154), welches zum absteigenden Ast gehört und im absteigenden Gefäß (153) mündet.

Da beim Drucken der Gefäße mit der Kapillartinte viele Zellen pro Tropfen verdruckt werden und nur die Zellen an den Randbereichen in der Lage sind sich mit dem umliegenden Gewebe zu vernetzen, müssen die überschüssigen Zellen ausgespült werden. Dazu und zur Versorgung der gedruckten Zellen wird nach jeder Fertigstellung eines Mikrokreislaufs, dieser mit Medium für eine kurze Zeit durchspült. Bei jedem Spülvorgang werden die unterhalb des letzten Mikrokreislauf liegenden Mikrokreisläufe mit gespült und die Zellen versorgt. Der Fluss des Mediums spült die unvernetzten Zellen aus. Horizontal verlaufende Gefäße sind dafür nicht optimal, weshalb die gedruckten Gefäße alle in einem Winkel von 10 bis 90 Grad zur Druckerplatte verlaufen.

Die auf- und absteigenden Gefäße (152 und 153), steigen in einem Winkel von 90° an. Die Gefäßfächer (155) können in einem Winkel von 10 - 90° ansteigen. Gedruckt wird bis zur Mitte des Einzelmoduls aufsteigend, danach absteigend, sodass die kleineren Gefäße immer aufsteigend zur Druckerplatte verlaufen. So können bei den unfertig gedruckten Gefäßen die überschüssigen Zellen gut ausgespült werden. Wenn ein Mikrokreislauf geschlossen ist, kann das Medium über das absteigende Gefäß ablaufen (Abb. und 15f).

Die Gefäßfächer werden in einem definierten Abstand gedruckt, wobei der Gefäßfächer des auf - und absteigenden Gefäßes abwechselnd untereinander verlaufen. Jeweils ein Gefäßfächer des aufsteigenden Gefäßes (152) und des absteigenden Gefäßes (153) werden durch Brückengefäße (156) miteinander verbunden und bilden den Mikrokreislauf (Abb. 15d). Dieser Vorgang wird so lange wiederholt, bis das Einzelmodul gefüllt ist. Zum Drucken eines Gewebes oder eines Organs, wird ein Gewebemodul (158) geduckt welches aus mehreren Einzelmodulen besteht. Die Einzelmodule werden auf der Druckerplatte, in ihrer Ausrichtung versetzt (Abb. 15g), bündig nebeneinander gedruckt, sodass sich ein Gewebemodul von definierter Seitenlänge und Dicke ergibt. Die Organmodule können zeitlich parallel auf verschiedenen Druckern gedruckt werden und mittels eines Klebers (z.B. Fibrinkleber) zu größeren Gewebe- oder Organeinheiten verbunden werden (Abb. 15e).

Um die Gewebemodule an den Kreislauf des Körpers anschließen zu können, muss bevorzugt ein Gewebeabschlussmodul (158) (Abb.15g) und ein Gewebeanschlussmodul (Abb.15h) gedruckt werden.

Das Gewebeabschlussmodul (158) besteht aus einem Gewebemodul, bei dem sich in den Einzelmodulen die auf- und absteigenden Gefäße nach oben verjüngen und so verschließen und mit mehreren Zelllagen (1510) abgeschlossen werden.

Das Anschlussmodul muss bevorzugt die einzelnen Gefäße der der Einzelmodule zu größeren Gefäßen zusammenführen und dabei die anatomischen und chirurgischen Vorgaben berücksichtigen. Dabei verlaufen die zu - und abführenden Gefäße auf unterschiedlichen Ebenen zu den Anschlussgefäßen hin. Die aufsteigenden Gefäße und die absteigenden Gefäße sind wieder durch Brückengefäße zur Versorgung des Gewebes miteinander verbunden.

### Abbildung 16

### Abb. 16: Durchspülung der entstehenden Kapillaren mit Medium:

Der Druckvorgang ist dabei so programmiert, dass in immer wiederkehrenden Zyklen nach dem Erreichen einer bestimmten Höhe des Gewebes ein kurzer Spülvorgang eingeleitet wird, um die überschüssigen und unvernetzten Zellen auszuspülen. Dieser Spülprozess kann in verschiedenen Ausführungsformen vorliegen. Bevorzugt wird nach einer definierten Anzahl an Druckvorgängen einmal der aufsteigende Ast gespült, dann nach einer weiteren definierten Anzahl an Druckvorgängen der absteigende Ast. Immer alternierend, bis das Gewebemodul fertig gedruckt ist. Durch den alternierenden Spülvorgang können die zu - und ablaufenden Gefäße gespült werden, ohne die Gefäße zu überlasten.

### Abbildung 17

### Abb. 17: Leber - Organmodul:

### Einzelmodule am Beispiel der Leber:

Die Leber besteht aus ca. 1 - 1,5 Millionen Leberläppchen mit einem Durchmesser von 1-2 mm. In die Leber enden zwei Gefäße, die Leberarterie zur Versorgung des Gewebes mit Sauerstoff und Nährstoffen und die Pfortader, die das Blut aus Magen und Darm mit den aufgenommenen Nährstoffen und Giftstoffen transportiert. Aus der Leber heraus gehen die Venen die sich zur Hohlvene vereinen und der Gallengang.

Das Einzelmodul wird hier aus den einzelnen hexagonalen Leberläppchen (171) (Abb.17a) mit einem Durchmesser von 2 mm gebildet. Die Leberläppchen sind nebeneinander in 5 Reihen angeordnet und jeweils 5 Reihen hoch (Abb. 17c). Durch die Anordnung der Leberläppchen in den Reihen, das jede zweite Reihe um ein halbes Leberläppchen versetzt ist, lassen sie sich bei der Bildung von Organmodulen verzahnen.

Die einzelnen hexagonalen Leberläppchen haben in jeder Ecke 3 Gefäße (Arterie (172), Pfortader (174), Gallengang) und in der Mitte die Vene (175). Alle 4 Gefäße bilden wieder einen Mikrokreislauf (Abb.17b).

Das Leberläppchen ist in jeweils zu den einzelnen Eckgefäßen in 6 Segmente (176) unterteilt, die von den Mikrokreisläufen durchströmt werden und einen Gefäßfächer (Abb.17d) bilden. Die beiden zuführenden Gefäße, die Arterie und die Pfortader laufen im Mikrokreislauf parallel zueinander zur Vene hin. Ihnen entgegen kommt aus der Mitte die Vene, sie verläuft unterhalb der zuführenden Gefäße, so bilden die drei Gefäße ein Dreieck. Die Arterie und die Pfortader sind wieder mit Gefäßbrücken mit der Vene verbunden und bilden einen Mikrokreislauf. Der Gallengang befindet sich in der Mitte des Gefäßdreiecks.

### Abbildung 18

### Übereinanderlagerung von einzelnen gedruckten Leber - Organmodulen:

### Abbildung 19:

### Abb. 19: Beispielhafter Aufbau eines Inkubators:

Der Gewebeinkubator besteht aus einer mediumgefüllten Wanne (191) mit einem aufzulegenden Deckel (192). Auf dem Boden der Wanne ist ein Fuß (193) fest installiert, auf dem ein baugleicher Druckertischfuß (25) wechselbar angebracht ist. Die zu- und abführenden Mediumschläuche (194) zum Druckertischfuß werden über Öffnungen im Inkubatorgefäß herangeführt. Auf die Zapfen des Druckertischfußes (ohne Silikonmembran) wird mittels Magnetkraft der Druckertisch samt Druckplatte, Druckfolie und gedrucktem Gewebe aufgesetzt.

### Abbildung 20 u. 21:

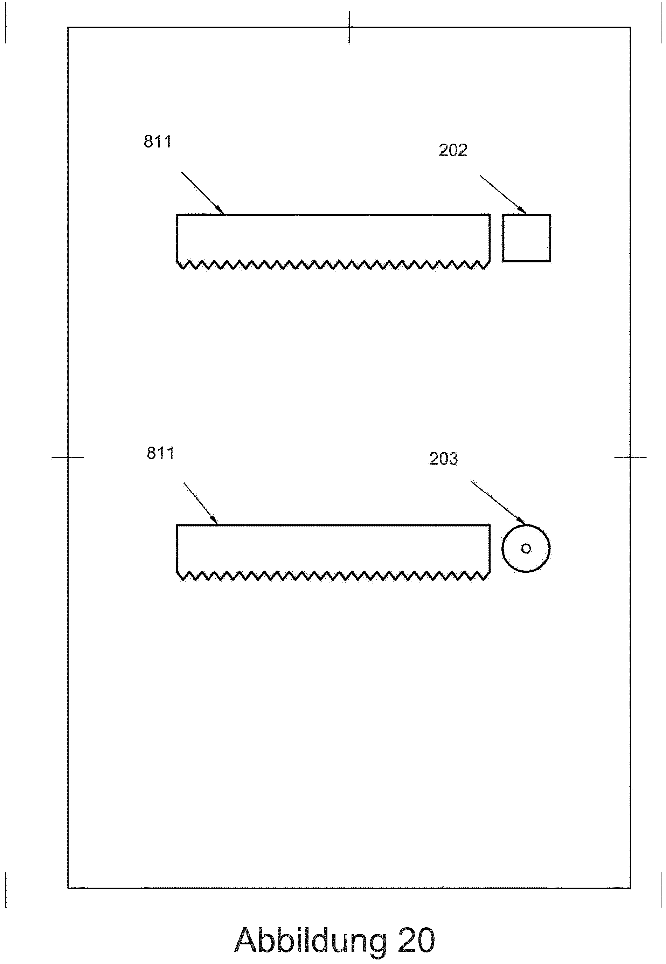

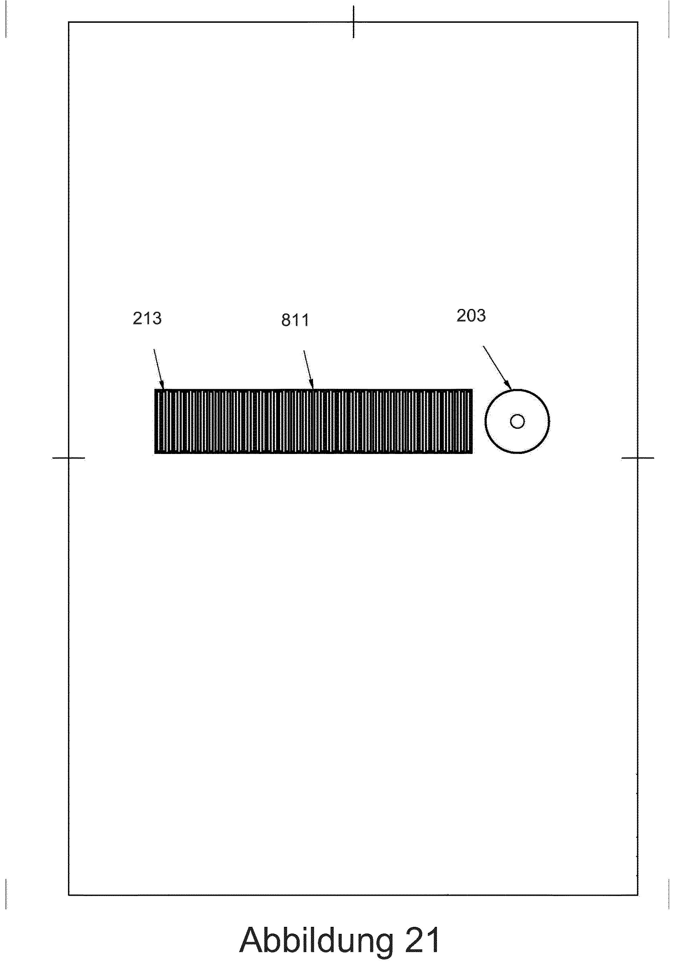

### Abb. 20 und 21: Beispielhafter Aufbau eines Druckerkopfes mit ungerichteter und gerichteter

### Quelle für elektomagnetische Strahlung.

### Bezugszeichen:

| | | | |
|---|---|---|---|
| 1 | Druckerkopfversorgungseinheit (1) | 174 | Pfortader |
| 2 | Druckerkopfversorgungseinheit (3) | 175 | Vene |
| 3 | Druckerkopfversorgungseinheit (4) | 176 | Segment |
| 4 | Druckerkopfversorgungseinheit (2) | 191 | Medium Tank |
| 5 | Druckerkopfversorgungseinheit | 192 | Deckel |
| 6 | Gehäuse | 193 | Fuß |
| 7 | Zentrale Steuereinheit des 3D-Druckers | 194 | Medium Zu- und Ablauf |
| 8 | Mediumtank | 202 | Ungerichtete elektromagnetische Strahlungsquelle |
| 9 | Medium zuführender Schlauch (2) | 203 | Gerichtete elektromagnetische Strahlungsquelle |
| 10 | Medium abführender Schlauch | 213 | Piezodüsenreihe |
| 11 | Abfall | | |
| 12 | Motor für den Druckertisch | | |
| 13 | Druckertisch | | |
| 14 | Medium qefüllter Druckraum | | |
| 15 | Piezodrucker | | |
| 21 | Medium Zu- und Ablauf | | |
| 22 | Mikrometerschraube | | |
| 23 | Schrittmotor | | |
| 24 | Magnetplatte | | |
| 25 | Druckertischfuß | | |
| 27 | Druckplatte | | |
| 28 | Bodenplatte für Druckraum | | |
| 29 | Druckraumwand (Rahmen) | | |
| 210 | Silikonmatte | | |
| 81 | Behälter für Tintenkonzentrat | | |
| 82 | Zelltank | | |
| 83 | Gehäuse | | |
| 84 | Pumpe | | |
| 85 | Pumpe (1) | | |
| 86 | Zellkonzentrator | | |
| 87 | Mischeinheit | | |
| 88 | Zellzähler (1) | | |
| 89 | Abführschlauch Medium | | |
| 810 | Zellzähler | | |
| 811 | Piezodruckerkopf | | |
| 812 | Tintenzuführunq | | |
| 151 | kubisches Einzelmodul | | |
| 152 | aufsteigende Gefäß | | |
| 153 | absteigende Gefäß | | |
| 154 | kleines Gefäß | | |
| 155 | Gefäßfächer | | |
| 156 | Brückengefäße | | |
| 158 | Gewebeabschlussmodul | | |
| 1510 | Zelllagen | | |
| 171 | Leberläppchen | | |
| 172 | Arterie | | |

## Patentansprüche

1. **3D-Druckverfahren** zur Herstellung von vaskulären Strukturen aufweisenden Geweben und Organen mittels
**(a)** eines Droplet-Druckers (15) für photorealistische hochauflösende Drucke und
**(b)** einer Vorrichtung zum Applizieren elektromagnetischer Wellen (202, 203) umfassend die Schritte:
- Bereitstellung mindestens einer Biotinte und einer Kapillartinte mit Zellen und Vernetzungsmolekülen in dem Droplet-Drucker (15)
- Aufbringen mindestens eines Tropfens der Biotinte und der Kapillartinte auf eine Reaktionsebene (13)
- in-Kontakt-bringen der elektromagnetischen Wellen mit den Vernetzungsmolekülen in diesen Tropfen auf der Reaktionsebene (13)
- und Aktivierung der Vernetzungsmoleküle mittels ungerichteter oder gerichteter Bewegungen der elektromagnetischen Wellen in den Tropfen, wodurch vernetzte Strukturen ausgebildet und so vaskuläre Strukturen erhalten werden,
**dadurch gekennzeichnet, dass** die Kapillartinten nur im Randbereich des Tropfens vernetzen oder schichtbildend reagieren und wobei unvernetzte oder ungebundene Bestandteile beseitigt werden.

2. Verfahren nach Anspruch 1,
**dadurch gekennzeichnet, dass**
die Tropfengröße zwischen 1 fl (Femtoliter) und 1 µl (Microliter) liegt.

3. Verfahren nach Anspruch 1 bis 2,
**dadurch gekennzeichnet, dass**
die Vernetzungs- oder schichtbildenden Reaktionen im Randbereich der Kapillartropfen durch Bestandteile in den Tinten oder durch den Einfluss von elektromagnetischen Wellen hervorgerufen werden.

4. Verfahren nach Anspruch 1 bis 3,
**dadurch gekennzeichnet, dass**
Reaktionen im Randbereich der Kapillartinten hervorgerufen werden durch:
- Thiol-en Reaktionen
- Cycloadditionen
- nucleophile Ringöffnungen
- Selfassembling von Molekülen und Partikeln
- selektive Faktoren an den Zellen der Kapillartinten und/oder Biotinten.

5. Verfahren nach Anspruch 1 bis 4,
**dadurch gekennzeichnet, dass**
die mindestens eine Biotinte Moleküle umfasst, die die Physiologie des gedruckten Gewebes oder Organs unterstützen.

6. Verfahren nach Anspruch 1 bis 5,
**dadurch gekennzeichnet, dass**
die mindestens eine Biotinte Wachstumsfaktoren, Transkriptionsfaktoren, Signalmoleküle, Markermoleküle und/oder Zielmoleküle für spätere Schlüssel-Schloss-Reaktionen enthält.

7. Verfahren nach Anspruch 1 bis 6,
**dadurch gekennzeichnet, dass**
die gedruckten vaskulären Strukturen während des Drucks mit Nährmedium oder Blut durchströmt werden.

8. Verfahren nach Anspruch 1 bis 7,
**dadurch gekennzeichnet, dass**
die Reaktionsebene in einem Bioreaktor positioniert ist, der insbesondere während oder nach Beendigung des Drucks mit einem Blutkreislauf verbunden vorliegt

9. Verfahren nach Anspruch 1 bis 8,
**dadurch gekennzeichnet, dass**
die Vorrichtung zum Applizieren der elektromagnetischen Wellen (202, 203) eine UV-Lampe, eine Diode, ein Screen oder eine Laserstrahlvorrichtung ist.

10. Eine Steuereinheit (7) für die Steuerung von Druckerkopfversorgungseinheiten (1, 2, 3, 4, 5), einem Motor (12) für einen Druckertisch (13), einer Vorrichtung zum Applizieren elektromagnetischer Wellen (202, 203) und einem Droplet-Drucker (15) für photorealistische hochauflösende Drucke und optional verschiedenen Druckern gemäß einem Druckmuster zum 3D-Druck von vaskulären Strukturen aufweisenden Geweben und Organen **dadurch gekennzeichnet, dass** folgende Eigenschaften im Druckmuster vorliegen:
- Gewebeebene des Druckmusters, die aus verschiedenen Tintentypen besteht, die **dadurch gekennzeichnet sind, dass** sie mittels des Droplet-Druckers (15) für photorealistische hochauflösende Drucke druckbar sind und mittels der Vorrichtung zum Applizieren elektromagnetischer Wellen (202, 203) vernetzbar sind, wobei einige von den verschiedenen Tintentypen nur im Randbereich eines Tropfens in Nachbarschaft zu anderen Tropfentypen eine Vernetzung oder Polymerisierung durchlaufen
- Gewebeebene aus verschiedenen, geordnet aufgebrachten Zelltypen, die von angeschnittenen, innen hohlen Gefäßen (152, 153, 154), einschließlich Venen, Arterien und Kapillaren, durchzogen sind
- Unterteilung größerer Gewebe oder Organe in Einzelmodule (151) und Vereinfachung des Druckbildes für einen ökonomichen Druckablauf
- Druck der Einzelmodule (151) unabhängig voneinander auf dem gleichen Droplet-Drucker (15) oder auf verschiedenen Druckern und anschließendes Verkleben der Einzelmodule (151) zum gewünschten Aufbau des Gewebes oder eines Organs
- Einzelmodule (151) mit mindestens einem aufsteigenden (152) und einem absteigenden Ast (153) für Mediumzuleitung und -ableitung sowie fächerförmig dazwischen liegenden Kapillaren, die einen Mikrokreislauf bilden, der die kleinste Gefäßeinheit bildet
- Vereinigung der Mikrokreisläufe zu Hauptgefäßen für Mediumzulauf und ablauf beim Aufbau größerer Strukturen aus Einzelmodulen (151)
- Stabilisierung des gedruckten Gewebes durch eine bioabbaubare oder resorbierbare Druckfolie, die in Z-Richtung beide Druckseiten begrenzt und die Neben- bzw. Hauptanschlüsse für die Gefäße besitzt.

11. Steuereinheit (7) gemäß dem vorherigen Anspruch umfassend einen spezifischen Algorithmus zur Berechnung des Druckmusters und zur Organisation eines
Druckvorgangs **dadurch gekennzeichnet dass** die Berechnung des Druckmusters in Bezug auf den verwendeten Zelltyp sowie in Bezug auf einen Abstand und eine Größe der Kapillaren und der arteriellen und venösen Strukturen erfolgt und dass einerseits eine optimale kapillare Versorgung sowie andererseits die anatomischen Gegebenheiten des jeweiligen Gewebaufbaus in einem gemeinsamen, modularen und ökonomisch strukturiertem Druckmuster vereinfacht werden, sowie
- dass der Algorithmus größere Zielstrukturen in kleinere Einzelmodule (151) zerlegt, die unabhängig voneinander gedruckt werden können, sowie dass
- der Algorithmus die Datei-Informationen zum 3D-Druck einer Druckplatte (27) und der Druckfolie liefert und diese aus dem Computerprogramm für andere 3D-Drucker exportierbar macht, sowie dass
- der Algorithmus zur Organisation des Druckvorganges folgende Informationen erhält und verarbeitet:
- Informationen über anatomische und physiologische Daten sowie die Maße der zu druckenden Gewebe und Organe,
- Informationen aus den Druckerkopfversorgungseinheiten (1, 2, 3, 4, 5) über eine Füllstände von Tanks (8, 82, 191), eine Zellkonzentration in einem Reaktor und in einem Zellkonzentrator (86) und an dessen Ausgang, die gemischten Volumina in einem Mixer (87), sowie Informationen über Vorgänge im Druckerkopf (811),
- Informationen über die Position des Druckertisches (13), eines Mediumfüllstandes im Druckraum (14), eines Mediumvolumenstroms pro Zeiteinheit in den Zu- und Ableitungen im Druckertischbereich, über eine Laufrichtung des Medium-Volumenstroms,
- Informationen über die derzeitigen Maße des gedruckten Gewebes,
- Informationen über die augenblickliche Leistung eines Druckertischmotors (12), sowie dass der Algorithmus Informationen über die Aktivität einer Quelle von elektromagnetischen Wellen (202, 203) erhält
- und dass der Algorithmus folgende Signale an die entsprechenden Druckerbereiche sendet:
- Signale an die einzelnen Komponenten der Druckerkopfversorgungseinheit (1, 2, 3, 4, 5) zum Mixen der erforderlichen Tintenbestandteile
- Signale an Pumpen (84, 85), die zum spezifischen Volumentransport der Tinten in den Mixer (87) und in die Druckerköpfe (811) dienen
- Signale zur Organisation des Zulaufs und Ablaufs für das Medium auf dem Weg vom Druckertischfuß (25) zu den Gefäßen der Gewebe sowie zur Organisation eines Spülvorgangs in den Kapillaren
- Signale zur erforderlichen Bewegung des Druckertisches (13)
- Signale an die Druckeröpfe (811) zur selektiven Ansteuerung von Druckerdüsen (213) und zur Fahrt eines Druckerkopfschlittens
- Signale an die Vorrichtung zum Applizieren der elektromagnetischen Wellen (202, 203)
- Signale für fakultative Druckpausen während der Kapillarspülung.

12. 3D Drucker umfassend eine Steuereinheit gemäß Anspruch 11 und eine Druckerkopfversorgungseinheit zum 3D-Druck von vaskulären Strukturen aufweisenden Geweben und Organen **dadurch gekennzeichnet, dass** die Druckerkopfversorgungseinheit aus folgenden Komponenten aufgebaut ist:
- einem Zellkonzentrator, in dem die gezüchteten Zellen aus dem Zelltank zu einer bestimmten Zellkonzentration aufkonzentriert werden
- einem Tintenkonzentrattank
- einer Mischeinheit mit Zellzähler, in der nach Vorgaben des Druckalgorithmus die erforderliche Zellzahl pro Volumen sowie die erforderliche Tintenkonzentration gemischt und zum Druckerkopf weitergeleitet wird,
- wobei die Anzahl der Druckerkopfversorgungseinheiten sich nach der Anzahl der benötigten Tinten richtet und variieren kann, und
- die Druckerkopfversorgungseinheit einerseits vom Algorithmus des Druckers gesteuert wird, um eine erforderliche Tintenzusammensetzung zu mischen, und sie andererseits eine Reihe von Sensoren besitzt, die dem Druckeralgorithmus Informationen über die Eigenschaften der gegenwärtig gemischten Tinten liefern, auf die der Algorithmus reagiert.

## Claims

1. 3D printing process for the production of tissues and organs with vascular structures organs by means of
(a) a droplet printer (15) for photorealistic high-resolution prints and
(b) a device for applying electromagnetic waves (202, 203) comprising the steps of:
- providing at least a bio-ink and a capillary ink comprising cells and crosslinking molecules in the droplet printer (15)
- applying at least one droplet of the bioink and the capillary ink to a reaction plane (13)
- bringing the electromagnetic waves into contact with the crosslinking molecules in these droplets on the reaction plane (13)
- and activating the cross-linking molecules by means of undirected or directed movements of the electromagnetic waves in the droplets, whereby cross-linked structures are formed and vascular structures are thus obtained,
**characterised in that** the capillary inks only crosslink or react in a layer-forming manner in the edge region of the drop and wherein uncrosslinked or unbound components are removed.

2. Process according to claim 1,
**characterised in that**
the droplet size is between 1 fl (femtolitre) and 1 µl (microlitre).

3. Process according to claim 1 to 2,
**characterised in that**
the cross-linking or layer-forming reactions in the edge region of the capillary droplets are caused by components in the inks or by the influence of electromagnetic waves.

4. Process according to claims 1 to 3,
**characterised in that**
reactions in the edge region of the capillary inks are caused by
- thiol-ene reactions
- cycloadditions
- nucleophilic ring openings
- self-assembling of molecules and particles
- selective factors on the cells of the capillary inks and/or bioinks.

5. Process according to claim 1 to 4,
**characterised in that**
the at least one bioink comprises molecules which support the physiology of the printed tissue or organ.

6. Process according to claim 1 to 5,
**characterised in that**
the at least one bioink contains growth factors, transcription factors, signalling molecules, marker molecules and/or target molecules for subsequent key-lock reactions.

7. Process according to claim 1 to 6,
**characterised in that**
the printed vascular structures are perfused with nutrient medium or blood during printing.

8. Process according to claims 1 to 7,
**characterised in that**
the reaction plane is positioned in a bioreactor, which in particular is connected to a blood circuit during or after the end of printing.

9. Process according to claim 1 to 8,
**characterised in that**
the device for applying the electromagnetic waves (202, 203) is a UV lamp,a diode, a screen or a laser beam device.

10. A control unit (7) for the control of printer head supply units (1, 2, 3, 4, 5),a motor (12) for a printer table (13), a device for applying electromagnetic waves (202, 203) and a droplet printer (15) for photorealistic high-resolution prints and optionally different printers according to a print pattern for 3D printing of tissues and organs having vascular structures, **characterised in that** the following properties are present in the print pattern:
- Tissue layer of the printed pattern consisting of different types of inks **characterised in that** they are printable by means of the droplet printer (15) for photorealistic prints and can be cross-linked by means of the device for applying electromagnetic waves (202, 203), wherein some of the different types of inks are ink types only undergo crosslinking or polymerisation in the edge region of a drop in the vicinity of other drop types
- Tissue layer consisting of different, orderly applied cell types which are cut, internally hollow vessels (152, 153, 154), including veins, arteries and capillaries
- Subdivision of larger tissues or organs into individual modules (151) and simplification of the print image for an economical printing process
- Printing the individual modules (151) independently of each other on the same droplet printer (15) or on different printers and subsequent bonding of the individual modules (151)to the desired structure of the tissue or an organ
- Individual modules (151) with at least one ascending (152) and one descending branch (153) for medium-supply and -drainage as well as fan-shaped interposed capillaries, which form a microcirculation forming the smallest vascular unit
- Combining the microcircuits into main vessels for medium-supply and -drainage when constructing larger structures from individual modules (151)
- Stabilisation of the printed tissue using a biodegradable or resorbable printing foil that borders both sides of the print in the Z-direction and has the secondary or main connections for the vessels.

11. Control unit (7) according to the preceding claim comprising a specific algorithm for calculating the print pattern and organising a printing process, **characterised in that** the calculation of the print pattern in relation to the cell type used and in relation to a distance and a size of the capillaries and of the arterial and venous structures, and **in that** an optimal capillary supply on the one hand and the anatomical conditions of the respective tissue structure in a common, modular and economically economically structured pressure pattern are simplified, and
- that the algorithm breaks down larger target structures into smaller individual modules (151), which can be printed independently of each other, and that
- the algorithm provides the file information for 3D printing a printing plate (27) and the printing foil and makes these exportable from the computer program for other 3D printers, and that
- the algorithm receives and processes the following information to organise the printing process:
- information on anatomical and physiological data and the dimensions of the tissues and organs to be printed,
- information from the printer head supply units (1, 2, 3, 4, 5) about the filling levels of tanks (8, 82), and filling levels of tanks (8, 82, 191), a cell concentration in a reactor and in a cell concentrator cell concentrator (86) and at its outlet, the mixed volumes in a mixer (87), as well as information about processes in the printer head (811),
- information about the position of the printer table (13), a medium filling level in the pressure chamber (14), a medium volume flow per unit of time in the supply and discharge lines in the the printer table area, about a running direction of the medium volume flow,
- information about the current dimensions of the printed tissue,
- information about the current power of a printer table motor (12), and that the algorithm provides information about the activity of a source of electromagnetic waves (202, 203)
- and that the algorithm sends the following signals to the corresponding printer areas:
- Signals to the individual components of the printer head supply unit (1, 2, 3, 4, 5) for mixing the required ink components
- signals to pumps (84, 85), which are used for the specific volume transport of the inks into the mixer (87) and into the printer heads (811)
- Signals for organising the supply and discharge of the medium on the way from the printer table base (25) to the vessels of the tissues as well as for organising a rinsing process in the capillaries
- Signals for the required movement of the printer table (13)
- Signals to the printer heads (811) for selective control of printer nozzles (213) and for the movement of a printer head carriage
- Signals to the device for applying the electromagnetic waves (202, 203)
- Signals for optional printing pauses during capillary rinsing.

12. 3D printer comprising a control unit according to claim 11 and a printer head supply unit printer head supply unit for 3D printing of tissues and organs having vascular structures organs, **characterised in that** the printer head supply unit is composed of the following components:
- a cell concentrator in which the cultured cells from the cell tank are concentrated to a specific cell concentration
- an ink concentrate tank
- a mixing unit with cell counter, in which the required number of cells per volume and the required number of cells per volume as well as the reqired ink concentration is mixed and is forwarded to the printer head,
- where the number of printer head supply units depends on the number of inks required and may vary, and
- the printer head supply unit is controlled by the algorithm of the printer on the one hand, to mix a required ink composition, and on the other hand it has a number of sensors which provide the printer algorithm with information about the characteristics of the inks currently mixed to which the algorithm responds.

## Revendications

1. **Procédé d'impression 3D** pour fabriquer des tissus et des organes dotés de structures vasculaires au moyen
**(a)** d'une imprimante à gouttelettes (15) pour des impressions photoréalistes à haute résolution et
**(b)** d'un dispositif pour appliquer des ondes électromagnétiques (202, 203) comprenant les étapes suivantes :
- fourniture d'au moins une encre biologique et une encre capillaire avec des cellules et des molécules de réticulation dans l'imprimante à gouttelettes (15),
- application d'au moins une goutte de l'encre biologique et de l'encre capillaire sur un plan de réaction (13),
- mise en contact des ondes électromagnétiques avec les molécules de réticulation de ces gouttes au niveau de la réaction (13),
- et activation des molécules de réticulation au moyen de mouvements non dirigés ou dirigés des ondes électromagnétiques dans les gouttes, grâce à quoi des structures réticulées sont formées et des structures vasculaires sont ainsi obtenues,
**caractérisé en ce que** les encres capillaires réticulent ou réagissent uniquement pour former une couche dans la zone marginale de la goutte et dans lequel les composants non réticulés ou non liés sont éliminés.

2. Procédé selon la revendication 1,
**caractérisé en ce que**
la taille des gouttes est comprise entre 1 fl (femtolitre) et 1 µl (microlitre).

3. Procédé selon la revendication 1 et 2,
**caractérisé en ce que**
les réactions de réticulation ou de formation de couches dans la zone marginale des gouttes capillaires sont provoquées par des composants présents dans les encres ou par l'influence des ondes électromagnétiques.

4. Procédé selon la revendication 1 à 3,
**caractérisé en ce que**
les réactions dans la zone marginale des encres capillaires sont provoquées par :
- des réactions thiol-ène ;
- des cycloadditions ;
- des ouvertures d'anneaux nucléophiles ;
- un autoassemblage de molécules et de particules ;
- des facteurs sélectifs sur les cellules des encres capillaires et/ou encres biologiques.

5. Procédé selon la revendication 1 à 4,
**caractérisé en ce que**
l'au moins une encre biologique comprend des molécules qui soutiennent la physiologie du tissu ou de l'organe imprimé.

6. Procédé selon la revendication 1 à 5,
**caractérisé en ce que**
l'au moins une encre biologique contient des facteurs de croissance, des facteurs de transcription, des molécules de signalisation, des molécules marqueurs et/ou des molécules cibles pour des réactions de type clef-serrure ultérieures.

7. Procédé selon la revendication 1 à 6,
**caractérisé en ce que**
les structures vasculaires imprimées sont perfusées avec un milieu nutritif ou du sang pendant l'impression.

8. Procédé selon la revendication 1 à 7,
**caractérisé en ce que**
le niveau de réaction est placé dans un bioréacteur qui est relié à une circulation sanguine, en particulier pendant ou après la fin de la pression.

9. Procédé selon la revendication 1 à 8,
**caractérisé en ce que**
le dispositif d'application des ondes électromagnétiques (202, 203) est une lampe UV, une diode, un écran ou un dispositif à faisceau laser.

10. Unité de commande (7) pour commander les unités d'alimentation de tête d'imprimante (1, 2, 3, 4, 5), un moteur (12) pour une table d'imprimante (13), un dispositif d'application d'ondes électromagnétiques (202, 203) et une imprimante à gouttelettes (15) pour des impressions photoréalistes à haute résolution et éventuellement diverses imprimantes selon un motif d'impression pour l'impression 3D de tissus et d'organes ayant des structures vasculaires, **caractérisée en ce que** les propriétés suivantes sont présentes dans le motif d'impression :
- plan tissulaire du motif d'impression, constitué de différents types d'encres, **caractérisés en ce qu'**ils sont imprimables à l'aide de l'imprimante à gouttelettes (15) pour des impressions photoréalistes à haute résolution et réticulables à l'aide du dispositif d'application d'ondes électromagnétiques (202, 203), dans laquelle certains des différents types d'encre ne subissent une réticulation ou une polymérisation que dans la zone marginale d'une goutte, à proximité d'autres types de gouttes,
- niveau tissulaire constitué de différents types de cellules ordonnées, traversées par des vaisseaux coupés et creux à l'intérieur (152, 153, 154), comprenant des veines, des artères et des capillaires,
- division de tissus ou d'organes plus grands en modules individuels (151) et simplification de l'image imprimée pour un processus d'impression économique,
- impression des modules individuels (151) indépendamment les uns des autres sur la même imprimante à gouttelettes (15) ou sur des imprimantes différentes, puis collage des modules individuels (151) pour former la structure souhaitée du tissu ou d'un organe,
- modules individuels (151) comportant au moins une branche montante (152) et une branche descendante (153) pour l'alimentation en fluide et l'évacuation de fluide, ainsi que des capillaires disposés en éventail entre elles, qui forment un microcircuit constituant la plus petite unité vasculaire,
- combinaison des microcircuits pour former des vaisseaux principaux pour l'entrée et la sortie de fluide lors de la construction de structures plus grandes à partir de modules individuels (151),
- stabilisation du tissu imprimé par un film d'impression biodégradable ou résorbable, qui délimite les deux faces d'impression dans la direction Z et présente les connexions secondaires ou principales pour les vaisseaux.

11. Unité de commande (7) selon la revendication précédente comprenant un algorithme spécifique pour calculer le motif d'impression et pour organiser un processus d'impression,
**caractérisée en ce que** le calcul du motif d'impression est lié au type de cellule utilisé et est effectué en rapport à une distance et une taille des capillaires et des structures artérielles et veineuses et que, d'une part, une alimentation capillaire optimale et, d'autre part, les conditions anatomiques de la structure tissulaire respective sont simplifiées dans un motif d'impression commun, modulaire et économiquement structuré, et
- que l'algorithme décompose les structures cibles plus grandes en modules individuels plus petits (151) qui peuvent être imprimés indépendamment les uns des autres, et que
- l'algorithme fournit les informations de fichier pour l'impression 3D d'une plaque d'impression (27) et du film d'impression et les rend exportables à partir du programme informatique pour d'autres imprimantes 3D, et que
- l'algorithme d'organisation du processus d'impression reçoit et traite les informations suivantes :
- des informations sur les données anatomiques et physiologiques ainsi que les dimensions des tissus et organes à imprimer,
- des informations provenant des unités d'alimentation de tête d'imprimante (1, 2, 3, 4, 5) concernant un niveau de remplissage des réservoirs (8, 82, 191), une concentration de cellules dans un réacteur et dans un concentrateur de cellules (86) et à sa sortie, les volumes mélangés dans un mélangeur (87), ainsi que des informations sur les processus dans la tête d'impression (811),
- des informations concernant la position de la table d'impression (13), un niveau de fluide dans la chambre d'impression (14), un débit volumique de fluide par unité de temps dans les conduites d'alimentation et d'évacuation dans la zone de la table d'impression, autour d'une direction du débit volumique de fluide,
- des informations sur les dimensions actuelles du tissu imprimé,
- des informations sur les performances actuelles d'un moteur de table d'imprimante (12), et que l'algorithme reçoit des informations sur l'activité d'une source d'ondes électromagnétiques (202, 203),
- et que l'algorithme envoie les signaux suivants aux zones d'imprimante correspondantes :
- des signaux aux différents composants de l'unité d'alimentation de la tête d'impression (1, 2, 3, 4, 5) pour mélanger les composants d'encre requis,
- des signaux aux pompes (84, 85), qui servent au transport volumique spécifique des encres dans le mélangeur (87) et dans les têtes d'imprimante (811),
- des signaux pour organiser l'entrée et la sortie du fluide sur le chemin entre le pied de table d'imprimante (25) et les vaisseaux des tissus et pour organiser un processus de rinçage dans les capillaires,
- des signaux pour le mouvement requis de la table de l'imprimante (13),
- des signaux aux têtes d'imprimante (811) pour la commande sélective des buses d'imprimante (213) et pour déplacer un chariot de tête d'imprimante,
- des signaux au dispositif d'application des ondes électromagnétiques (202, 203),
- des signaux pour les coupures de pression facultatives pendant le rinçage des capillaires.

12. Imprimante 3D comprenant une unité de commande selon la revendication 11 et une unité d'alimentation de tête d'imprimante pour l'impression 3D de tissus et d'organes ayant des structures vasculaires, **caractérisée en ce que** l'unité d'alimentation de tête d'imprimante est construite à partir des composants suivants :
- un concentrateur de cellules dans lequel les cellules cultivées du réservoir de cellules sont concentrées à une concentration cellulaire spécifique,
- un réservoir de concentré d'encre,
- une unité de mélange avec un compteur de cellules dans laquelle le nombre requis de cellules par volume et la concentration d'encre requise sont mélangés selon les spécifications de l'algorithme d'impression et transmis à la tête d'impression,
- dans laquelle le nombre d'unités d'alimentation de tête d'imprimante dépend du nombre d'encres requises et peut varier, et
- l'unité d'alimentation de tête d'imprimante est, d'une part, commandée par l'algorithme de l'imprimante pour mélanger une composition d'encre requise et, d'autre part, comporte une série de capteurs qui fournissent à l'algorithme de l'imprimante des informations sur les propriétés des encres actuellement mélangées auquel l'algorithme réagit.
